# EUROPEAN PATENT APPLICATION

(11) **EP 4 692 127 A1**
(43) Date of publication of application: **11.02.2026**
(21) Application number: 24778261.8
(22) Date of filing: 29.03.2024
(51) Int. Cl.: C07K 16/32, C07K 19/00, C12N 15/81, A61K 39/395, A61P 35/00, A61P 35/02, G01N 33/574

(54) **SINGLE-CHAIN ANTIBODY AND IN VITRO SYNTHESIS SYSTEM AND USE THEREOF**

(30) Priority: 31.03.2023 CN 202310345905
(71) Applicant: Kangma-Healthcode (Shanghai) Biotech Co., Ltd, Pudong New Area Shanghai 201321 (CN)
(72) Inventor: GUO, Min, Shanghai 201321 (CN); YANG, Longjin, Shanghai 201321 (CN); XU, Liqiong, Shanghai 201321 (CN); HU, Jiabao, Shanghai 201321 (CN); ZHU, Yajun, Shanghai 201321 (CN); YU, Xue, Shanghai 201321 (CN)
(74) Representative: Dai, Simin
(86) International application number: PCT/CN2024/084959
(87) International publication number: WO 2024/199476

(57) **Abstract**

The present invention provides a single-chain antibody and an in vitro synthesis system and use thereof. The antibody has a high expression efficiency, high affinity and high stability, is high-efficiently expressed in one step in an in vitro cell-free synthesis system without secreting and expressing two different protein fragments of a light chain and a heavy chain, respectively, which is not easy to form a homodimer, has a relatively high expression efficiency, can be produced on a large scale, and has a good prospect of commercialization.

## Description

### TECHNICAL FIELD

The present disclosure belongs to the field of biotechnology, and specifically relates to a single-chain antibody and an *in vitro* synthesis system and use thereof.

### BACKGROUND

With the deepening understanding of the mechanisms of tumor occurrence and development, tumor therapy has entered the era of personalized treatment. In the era of personalized treatment, the most important therapeutic concept is targeted therapy. Tumor molecular targeted therapy utilizes specific genes or gene expression products that are expressed by tumor cells but rarely or not expressed by normal cells as therapeutic targets to maximize the killing of tumor cells. Antibodies exhibit high specificity for corresponding antigens. Specific antibodies can be prepared against particular target molecules (antigens) related to the occurrence and development of diseases. Antibodies show great potential and application prospects in treating various diseases, particularly in treating cancer, autoimmune diseases, and viral infections.

Based on the size of antibody molecules, recombinant antibodies can be classified into small molecular antibodies, multivalent antibodies, full-length antibodies, antibody genomic libraries, *etc.* Small molecular antibodies are antibody fragments with low molecular weight, prepared using prokaryotic or eukaryotic expression systems, primarily comprising the variable regions of light and heavy chains, and may or may not contain exogenous peptide chains. Typically, their size is only 1/3 or 1/2 of a full-length IgG molecule or even smaller, hence they are referred to as small molecule antibodies. Compared to full-length antibodies, small molecule antibodies offer advantages such as low molecular weight, ease of *in vitro* expression, and convenience for genetic engineering modifications to enhance antibody properties, thereby exhibiting broader application prospects. Small molecule genetically engineered antibodies mainly include single-chain variable fragments (scFv), bispecific antibodies (BsAb), triabodies, single-domain antibodies (VHH), single-chain disulfide-bond variable fragments (dsFv), antigen-binding fragments (Fab), and antibody F(ab')₂ fragments (Hoogenboom HR. Selecting and screening recombinant antibody libraries. Nat Biotechnol. 2005 Sep; 23(9): 1105-16). Currently, the most extensively studied members of the small molecule genetically engineered antibody family are single-chain antibodies, antibody Fab fragments, disulfide-bond antibodies, and single-domain antibodies.

The advantages of small molecule antibodies are as follows: (1) The preparation method is simpler compared to other genetically engineered antibodies. (2) The immunogenicity is much weaker than that of the original. If reshaped antibodies are constructed into small molecule antibodies, it is more likely to eliminate their immunogenicity. (3) Due to their low molecular weight, small molecule antibodies can more easily pass through blood vessel walls (Bitencourt ALB, Campos RM, Cline EN, Klein WL, Sebollela A. Antibody Fragments as Tools for Elucidating Structure-Toxicity Relationships and for Diagnostic/Therapeutic Targeting of Neurotoxic Amyloid Oligomers. Int J Mol Sci. 2020 Nov 24; 21(23): 8920) and penetrate solid tumors, which is beneficial for tumor treatment. (4) Due to the absence of an Fc segment, they do not produce Fc-mediated CDC and ADCC toxic side effects. Compared to full-length antibodies, they have a shorter half-life and a faster turnover *in vivo* (Kholodenko RV, Kalinovsky DV, Doronin II, Ponomarev ED, Kholodenko IV. Antibody Fragments as Potential Biopharmaceuticals for Cancer Therapy: Success and Limitations. Curr Med Chem. 2019; 26(3): 396-426), which is beneficial for radioimmunoimaging to detect tumors; they cannot bind to receptors on non-target cells, allowing more concentrated delivery to tumor sites. (5) Their small size enables them to bind to antigens distributed in the surface grooves of the virus, which is beneficial for the treatment of viral diseases. (6) By attaching appropriate enzyme genes or toxin protein genes to the ends of small molecule antibody genes, enzyme-linked antibodies or immunotoxins can be produced in large quantities. (7) They can be expressed in microbial systems, allowing for fermentative production of antibodies with faster production and higher yields, thereby reducing costs (Fernandes JC. Therapeutic application of antibody fragments in autoimmune diseases: current state and prospects. Drug Discov Today. 2018 Dec; 23(12): 1996-2002), making antibody therapy more accessible.

Traditional small molecule recombinant antibodies, such as scFv and Fab, have some issues: First, since scFv lacks the CH1 and CL fragments of Fab, most scFvs have lower affinity compared to their homologous Fab fragments. Additionally, scFv tends to form dimeric structures, so during long-term storage, the stability of scFv is poorer than that of the corresponding Fab (Hust M, Jostock T, Menzel C, Voedisch B, Mohr A, Brenneis M, Kirsch MI, Meier D, Dübel S. Single chain Fab (scFab) fragment. BMC Biotechnol. 2007 Mar 8; 7:14). Second, since Fab requires the separate secretion and expression of two different protein fragments, its processing and folding in the periplasm of *E. coli* involve connection via disulfide bonds. The molecular weight of Fab is approximately twice that of scFv, and the light chain of Fab tends to form homodimers after expression. Therefore, the secretion and expression efficiency of Fab in the *E. coli* system is lower than that of scFv. These shortcomings limit the commercial application of antibodies. To overcome their shortcomings, it is desirable to prepare novel antibodies that possess high expression efficiency, high affinity, and high stability.

In recent years, a novel antibody single-chain Fab (scFab) has been reported, which consists of an antibody heavy chain Fd and an antibody light chain κ connected via a linker, expressing the protein in the form of a single-chain antibody (Koerber JT, Hornsby MJ, Wells JA. An improved single-chain Fab platform for efficient display and recombinant expression. J Mol Biol. 2015 Jan 30; 427(2): 576-86; Hanna R, Cardarelli L, Patel N, Blazer LL, Adams JJ, Sidhu SS. A phage-displayed single-chain Fab library optimized for rapid production of single-chain IgGs. Protein Science. 2020 August; 29(10): 2075-2084; Koerber JT, Hornsby MJ, Wells JA. An improved single-chain Fab platform for efficient display and recombinant expression. J Mol Biol. 2015 Jan 30; 427(2): 576-86).

The antibody single-chain Fab (scFab) integrates the advantages of Fab and scFv. Currently, the reported single-chain Fab exhibits the following characteristics: first, it demonstrates antigen-binding ability equivalent to that of Fab (Thie H, Binius S, Schirrmann T, Hust M, Dübel S. Multimerization domains for antibody phage display and antibody production. N Biotechnol. 2009 Dec 31; 26(6): 314-21); second, it exhibits higher expression efficiency compared to Fab, allowing for large-scale production of antibodies in the form of inclusion bodies (Bird RE, Hardman KD, Jacobson JW, Johnson S, Kaufman BM, Lee SM, Lee T, Pope SH, Riordan GS, Whitlow M. Single-chain antigen-binding proteins. Science. 1988 Oct 21; 242(4877): 423-6); third, antibodies can be produced in large quantities in *E. coli* expression systems or yeast eukaryotic expression systems (Jordan E, Al-Halabi L, Schirrmann T, Hust M, Dübel S. Production of single chain Fab (scFab) fragments in Bacillus megaterium. Microb Cell Fact. 2007 Nov 27; 6:38); scFab can replace scFv and Fab in phage vector display for screening highly active antibodies (Walker LM, Bowley DR, Burton DR. Efficient recovery of high-affinity antibodies from a single-chain Fab yeast display library. J Mol Biol. 2009 Jun 5; 389(2): 365-75). Sixth, scFab contains the antibody constant regions CH1 and CL, with a molecular weight approximately twice that of scFv, resulting in stronger van der Waals forces for the interaction between antibody molecules and greater stability compared to scFv (Hanna R, Cardarelli L, Patel N, Blazer LL, Adams JJ, Sidhu SS. A phage-displayed single-chain Fab library optimized for rapid production of single-chain IgGs. Protein Science. 2020 August; 29(10): 2075-2084).

Large-scale production of antibodies relies on the development of high-level production processes, which require advantages such as high expression, high stability, and scalability. Large-scale production of antibodies is a critical step in the industrialization and commercialization of antibody drugs and is also a major bottleneck restricting the development of the antibody industry in China.

### SUMMARY

To overcome the limitations of small molecule antibodies with poor stability and low secretion and expression efficiency of traditional commercially available monoclonal antibodies, which hinder the industrialization and commercial application of antibody drugs. The present disclosure provides a single-chain antibody and an *in vitro* synthesis method and use thereof, wherein the antibody possesses high expression efficiency, high affinity, and high stability, thereby exhibiting good prospects for industrialization and commercialization.

A first aspect of the present disclosure provides a single-chain antibody, wherein the single-chain antibody is formed by connecting a light chain or light chain fragment of an antibody to a heavy chain or heavy chain fragment of an antibody via a linker.

Specifically, the binding activity of the full-length single-chain antibody to the corresponding antigen is comparable to that of the commercially available full-length antibody.

Studies have found that single-chain antibodies prepared with linkers of different compositions and lengths exhibit varying affinity and expression activities. Therefore, the single-chain antibodies provided by the present disclosure have been optimized and improved in terms of the amino acid residues and their number in the linker, achieving both high-efficiency expression and high affinity of the single-chain antibodies.

To balance expression efficiency and specific affinity activity, the linker comprises at least 20 amino acid residues, preferably 25 to 180 amino acid residues, and more preferably 30 to 120 amino acid residues;

The amino acid comprised in the linker is selected from serine (S), glycine (G), glutamic acid (E), threonine (T), alanine (A), arginine (R), tyrosine (Y), isoleucine (I), lysine (K), leucine (L), glutamine (Q), histidine (H), phenylalanine (F), valine (V), proline (P), aspartic acid (D), methionine (M), and asparagine (N).

Preferably, the amino acids comprised in the linker are selected from serine (S), glycine (G), glutamic acid (E), threonine (T), and alanine (A).

In a preferred example, the total number of serine (S), glycine (G), glutamic acid (E), threonine (T), and alanine (A) comprised in the linker accounts for 100%, at least 99%, at least 98%, at least 97%, at least 96%, at least 95%, at least 90%, at least 85%, at least 80%, at least 75%, at least 70%, at least 65%, at least 60%, at least 55%, and at least 50% of the total number of amino acids in the linker.

In another preferred embodiment, the total number of serine (S) and glycine (G) comprised in the linker accounts for 100%, at least 99%, at least 98%, at least 97%, at least 96%, at least 95%, at least 90%, at least 85%, at least 80%, at least 75%, at least 70%, at least 65%, at least 60%, at least 55%, and at least 50% of the total number of amino acids in the linker.

The binding activity of the single-chain antibody to the corresponding antigen is comparable to that of the full-length antibody. Furthermore, the comparable activity refers to biological comparability, *e.g*., the binding activity of the single-chain antibody to the corresponding antigen reaches 0.1% to 10000%, 1% to 1000%, or 10% to 100% of the activity value of the full-length antibody, which is 85% to 115%, preferably 90% to 100%, and more preferably 95% to 100% in accordance with the present disclosure; the activity value may be any value expressing antibody activity in the art, such as an EC50 value.

The single-chain antibody may be synthesized in one step in an *in vitro* cell-free synthesis system, eliminating the need for separate secretion and expression of two different protein fragments, *i.e.,* the Fab light chain and heavy chain, which is less prone to forming homodimers, and exhibits higher expression efficiency and affinity activity compared to Fab. Moreover, it contains the antibody constant regions CH1 and CL, with a molecular weight approximately twice that of scFv, resulting in stronger van der Waals forces for the interaction between antibody molecules and greater stability compared to scFv.

Furthermore, the antibody is selected from IgM, IgG, IgA, IgD, and/or IgE;
further preferably, the IgG antibody is selected from IgG1, IgG2, IgG3 and/or IgG4.

In a preferred embodiment, the antibody is selected from an IgG1 antibody; preferably, the IgG1 antibody is Trastuzumab, Bevacizumab, Daratumumab, Omalizumab, Cetuximab, Adalimumab, Ustekinumab, Ocrelizumab, Infliximab, Envafolimab, Atlizumab, and/or Atezolizumab;
the IgG2 antibody is Denosumab, Erenumab, Tremelimumab, or Evocumab.

The antibody light chain is selected from a κ chain and/or a λ chain; the antibody light chain fragment is selected from a κ chain fragment and a λ chain fragment; the heavy chain is selected from a µ chain, a γ chain, an α chain, a δ chain, and/or an ε chain; the heavy chain fragment is selected from a µ chain fragment, a γ chain fragment, an α chain fragment, a δ chain fragment, and/or an ε chain fragment.

In a preferred embodiment, the antibody light chain is derived from Tislelizumab light chain TisLC or a fragment thereof, and the antibody heavy chain is derived from Tislelizumab heavy chain TisHC or a fragment TisFd thereof.

In a preferred embodiment, the antibody light chain is derived from Dupilumab light chain DupLC or a fragment thereof, and the antibody heavy chain is derived from Dupilumab heavy chain DupHC or a fragment DupFd thereof.

In a preferred embodiment, the antibody light chain is derived from Adalimumab light chain AdaLC or a fragment thereof, and the antibody heavy chain is derived from Adalimumab heavy chain AdaHC or a fragment AdaFd thereof.

In a preferred embodiment, the antibody light chain is derived from Bevacizumab light chain BevLC or a fragment thereof, and the antibody heavy chain is derived from Bevacizumab heavy chain BevHC or a fragment BevFd thereof.

In a preferred embodiment, the antibody light chain is derived from Daratumumab light chain DarLC or a fragment thereof, and the antibody heavy chain is derived from Daratumumab heavy chain DarHC or a fragment DarFd thereof.

In a preferred embodiment, the antibody light chain is derived from Omalizumab light chain OmaLC or a fragment thereof, and the antibody heavy chain is derived from Omalizumab heavy chain OmaHC or a fragment OmaFd thereof.

In a preferred embodiment, the antibody light chain is derived from Cetuximab light chain CetLC or a fragment thereof, and the antibody heavy chain is derived from Cetuximab heavy chain CetHC or a fragment CetFd thereof.

In a preferred embodiment, the antibody light chain is derived from Denosumab light chain DenLC or a fragment thereof, and the antibody heavy chain is derived from Denosumab heavy chain DenHC or a fragment DenFd thereof.

In a preferred embodiment, the antibody light chain is derived from Trastuzumab light chain TraLC or a fragment thereof, and the antibody heavy chain is derived from Trastuzumab heavy chain TraHC or a fragment thereof; in another preferred embodiment, the antibody light chain is derived from Trastuzumab light chain TraLC, and the antibody heavy chain fragment is a Trastuzumab heavy chain fragment TraFd.

Furthermore, the single-chain antibody further comprises a fluorescent signal peptide SP; preferably, the single-chain antibody is connected at the N-terminus of the light chain or light chain fragment with the signal peptide SP to improve the expression efficiency and expression level of the single-chain antibody.

Furthermore, the single-chain antibody further comprises a fluorescent protein tag; preferably, the single-chain antibody is connected at the C-terminus of the heavy chain or heavy chain fragment with the fluorescent protein tag; further preferably, the fluorescent protein tag is EGFP.

Furthermore, the single-chain antibody further comprises a tag label; preferably, the single-chain antibody is connected at the N-terminus of the light chain or light chain fragment with the tag label; preferably, the tag label added at the N-terminus of the light chain or light chain fragment is a His tag.

Furthermore, preferably, the fluorescent protein tag, the tag label, or the signal peptide is connected to the light chain, light chain fragment, heavy chain, or heavy chain fragment of the antibody via a single bond or a flexible linker; preferably, the flexible linker comprises 1 to 20 amino acid residues.

A second aspect of the present disclosure provides one or more isolated nucleic acids encoding the antigen-binding ligand protein provided in the first aspect.

Furthermore, the nucleic acid is a DNA sequence and/or an RNA sequence.

In a feasible embodiment, the nucleic acid is a linear DNA and is a PCR linear fragment.

Furthermore, the PCR linear fragment may be obtained by amplification techniques, which are not particularly limited, including, but not limited to, PCR amplification, isothermal amplification, constant-temperature amplification, and room-temperature amplification. Specifically, the isothermal amplification is preferably constant-temperature amplification. Commercially available DNA amplification systems may be employed, including, but not limited to, those provided by Biocompare, Neta Scientific Inc, ABM, Thermo Fisher Scientific, Expedeon, and Vivantis.

In another feasible embodiment, the nucleic acid is an RNA, which comprises an amino acid sequence encoding a single-chain antibody.

Furthermore, the RNA is prepared using *in vitro* nucleic acid amplification techniques. The *in vitro* nucleic acid amplification techniques that may be employed are not particularly limited, including, but not limited to, polymerase chain reaction (PCR) amplification, isothermal amplification, constant-temperature amplification, and room-temperature amplification. Specifically, the nucleic acid isothermal amplification methods applicable to the technical means of the present disclosure include, but are not limited to, loop-mediated isothermal amplification (LAMP), strand displacement amplification (SDA), nucleic acid sequence-based amplification (NASBA), rolling circle amplification (RCA), nicking enzyme-mediated isothermal amplification, helicase-dependent amplification (HDA), transcription-dependent amplification, hybrid capture, transcription-mediated amplification (TMA), recombinase-aided amplification (RAA), and recombinase polymerase amplification (RPA), preferably loop-mediated isothermal amplification.

A third aspect of the present disclosure provides one or more expression vectors, comprising a nucleic acid encoding the single-chain antibody provided in the first aspect.

Furthermore, the expression vector includes, but is not limited to, a eukaryotic plasmid vector, a eukaryotic viral vector, a prokaryotic plasmid, a specific vector, a shuttle vector, a minichromosome, and various vectors.

Preferably, the expression vector is a eukaryotic plasmid expression vector and a prokaryotic plasmid expression vector.

In a feasible embodiment, the expression vector is a circular DNA, further preferably a plasmid DNA, such as a pET series plasmid and a pGEM series plasmid.

A fourth aspect of the present disclosure provides a host cell, wherein the host cell contains the vector according to the third aspect of the present disclosure, or has the polynucleotide according to the second aspect integrated into its genome, or expresses the recombinant protein according to the first aspect.

In a preferred embodiment, the host cell is selected from a prokaryotic host cell and a eukaryotic host cell.

In another preferred embodiment, the host cell is a prokaryotic cell, such as *E. coli.*

In another preferred embodiment, the host cell is a eukaryotic cell, such as yeast, a progenitor cell, a Chinese hamster ovary cell, an insect cell, a wheat germ cell, and a rabbit reticulocyte.

Preferably, the yeast is one or more selected from the group consisting of *Saccharomyces cerevisiae* and one or more yeasts of the *Kluyveromyces,* in another preferred embodiment, the *Kluyveromyces* is one or more selected from the group consisting of *Kluyveromyces lactis, Kluyveromyces marxianus,* and *Kluyveromyces dobzhanskii.*

A fifth aspect of the present disclosure provides an *in vitro* cell-free protein synthesis system, comprising the nucleic acid molecule provided in the second aspect or the expression vector provided in the third aspect, or a lysate or extract of the host cell according to the fourth aspect of the present disclosure.

The *in vitro* cell-free protein synthesis system includes, but is not limited to, an *E. coli in vitro* protein synthesis system, a bacterial *in vitro* protein synthesis system, a mammalian cell *(e.g.,* HF9, Hela, CHO, HEK293) *in vitro* protein synthesis system, a plant cell *in vitro* protein synthesis system, a yeast cell *in vitro* protein synthesis system, and an insect cell *in vitro* protein synthesis system. It is preferably a yeast *in vitro* protein synthesis system, more preferably a *Kluyveromyces in vitro* protein synthesis system, and most preferably a *Kluyveromyces lactis* (*K. lactis) in vitro* protein synthesis system.

Furthermore, the *in vitro* protein synthesis system provided by the present disclosure includes, but is not limited to, (a) a cell extract, (b) optionally polyethylene glycol; (c) optionally exogenous sucrose, and (d) optionally a solvent.

Furthermore, the reaction system further comprises: tris(hydroxymethyl)aminomethane, potassium acetate, magnesium acetate, a mixture of nucleoside triphosphates (NTPs), a mixture of amino acids, *etc.*

The cell extract is typically used to provide substances such as ribosomes, transfer RNA (tRNA), aminoacyl-tRNA synthetases, initiation factors and elongation factors required for protein synthesis, and termination release factors, and may also endogenously provide other substances such as polymerases (RNA polymerase and/or DNA polymerase) after strain modification.

The protein components required in the *in vitro* cell-free protein synthesis system (*e.g.,* RNA polymerase) may be provided endogenously or added exogenously. When provided endogenously, reference may be made to the genetic modification methods provided in existing literature including, but not limited to, CN108690139A, CN109423496A, CN106978439A, CN110408635A, CN110551700A, CN110093284A, CN110845622A, CN110938649A, CN111378708A, CN111484998A, *"*Molecular and Cellular Biology, 1990, 10(1): 353-360", and their cited references. Examples of the methods include, but are not limited to, inserting the coding sequence into an intracellular free plasmid, integrating the coding gene into the cell genome, and a combination thereof. When provided exogenously, the amount may be controlled and adjusted according to the requirements of the system.

In some preferred embodiments, the yeast cell extract is a *Kluyveromyces cell* extract, *a Saccharomyces cerevisiae* cell extract, or a combination thereof. Specifically, in some preferred embodiments, the *Kluyveromyces* is *Kluyveromyces lactis (K. lactis).*

The *in vitro* protein synthesis systems, templates, plasmids, target proteins, *in vitro* protein synthesis reactions (incubation reactions), various preparation methods, various detection methods as technical elements of the present disclosure may also be independently selected from the suitable embodiments or implementation methods disclosed in the following references, including, but not limited to, CN111484998A, CN106978349A, CN108535489A, CN108690139A, CN108949801A, CN108642076A, CN109022478A, CN109423496A, CN109423497A, CN109423509A, CN109837293A, CN109971783A, CN109988801A, CN109971775A, CN110093284A, CN110408635A, CN110408636A, CN110551745A, CN110551700A, CN110551785A, CN110819647A, CN110845622A, CN110938649A, and CN110964736A. Unless conflicting with the purpose of the present disclosure, these references and their cited references are incorporated by reference in their entirety and for all purposes.

A sixth aspect of the present disclosure provides a method for detecting the activity of a single-chain antibody, wherein the activity of the single-chain antibody provided in the first aspect of the present disclosure is detected by enzyme-linked immunosorbent assay (ELISA). Furthermore, the ELISA is an indirect antibody assay or a competitive antibody assay.

Preferably, the indirect antibody assay by ELISA comprises the following steps:

The specific detection steps comprise: (1) binding a specific antigen to a solid-phase carrier to form a solid-phase antigen, and washing to remove unbound antigens and impurities; (2) adding diluted serum to be tested, wherein the specific antibody binds to the antigen to form a solid-phase antigen-antibody complex; after washing, only the specific antibody remains on the solid-phase carrier, while other antibodies and impurities in the serum that cannot bind to the solid-phase antigen are removed during the washing process; (3) adding an enzyme-labeled secondary antibody, which binds to the antibody in the solid-phase complex, thereby indirectly labeling the antibody with an enzyme; after washing, the amount of enzyme on the solid-phase carrier represents the amount of the specific antibody; for example, in detecting an antibody against a certain disease in humans, an enzyme-labeled goat anti-human IgG antibody may be used; (4) adding a substrate for color development, wherein the depth of color represents the amount of the antibody to be tested in the sample.

A seventh aspect of the present disclosure provides a use of the single-chain antibody provided in the first aspect of the present disclosure or the single-chain antibody prepared by the synthesis system provided in the fourth aspect for the diagnosis, prevention, and treatment of a disease or in the manufacture of a substance for the diagnosis, prevention, and treatment of a disease.

Furthermore, the disease is a tumor and/or cancer, and examples of tumors and/or cancers include, but are not limited to, lung cancer, breast cancer, esophageal cancer, gastric cancer, cervical cancer, colon cancer, rhabdomyosarcoma, liposarcoma, osteosarcoma, lymphoma, leukemia, liver cancer, cervical cancer, ovarian cancer, breast cancer, and other diseases.

Preferably, the disease is HER2-positive breast cancer and gastric cancer.

The advantageous effects of the present disclosure are as follows:
1) The present disclosure provides a single-chain antibody that can be efficiently expressed in a yeast cell-free system (*e*.*g*., D2P system) with a short production cycle, in which the light chain or light chain fragment (*e*.*g*., Trastuzumab light chain) and the heavy chain or heavy chain fragment (*e*.*g*., Trastuzumab heavy chain TraHC or heavy chain fragment TraFd) of an antibody are connected via a linker for expression.
2) The single-chain antibody provided by the present disclosure exhibits ErbB2 antigen-binding activity comparable to that of the commercially available monoclonal antibody Trastuzumab. Compared to the commercially available monoclonal antibody Trastuzumab (natural double-chain structure), the single-chain antibody has unparalleled ease of expression. Therefore, while ensuring antibody activity, the single-chain antibody provided by the present disclosure addresses the drawbacks of low expression efficiency in traditional antibodies and limitations that hinder their commercial application.
3) The single-chain antibody provided by the present disclosure, in which the light chain and heavy chain are connected via a linker, is less prone to aggregation and exhibits improved stability.
4) The single-chain antibody provided by the present disclosure possesses high expression efficiency, high affinity, and high stability. It can be produced on a large scale in combination with an *in vitro* cell-free protein synthesis system and specifically binds to the extracellular domain IV of ErbB2. Moreover, it has promising application prospects for the treatment of HER2-positive breast cancer and gastric cancer and exhibits favorable commercial prospects.

### BRIEF DESCRIPTION OF THE DRAWINGS

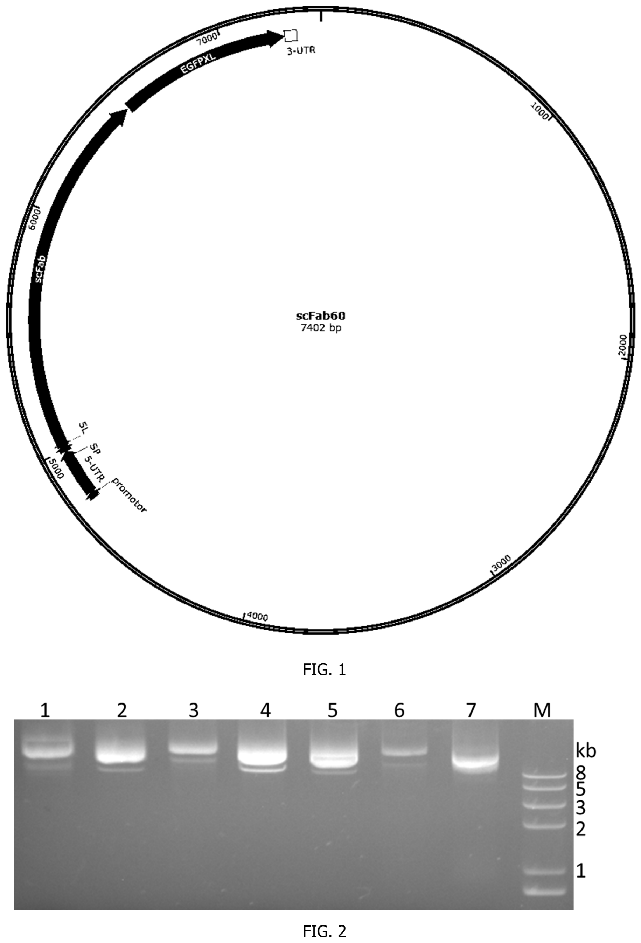

FIG. 1 shows the plasmid structure in Example 1 of the present disclosure.
   Promoter represents the promoter, 5-UTR represents the 5' untranslated region, SP represents the leader peptide, 5L represents the linker, the portion between SP and 5L represents the His tag, TrascFab represents the TrascFab proteins with different linker lengths, EGFP represents the enhanced green fluorescent protein sequence, and 3-UTR represents the 3' untranslated region and terminator.
FIG. 2 shows the electrophoretic analysis of the plasmids prepared in Examples 2 and 7 of the present disclosure, in which M represents the marker, and lanes 1 to 7 represent the plasmids TrascFab0, TrascFab6, TrascFab34, TrascFab60, TrascFab82, TrascFab120, and TrascIgG60, respectively.

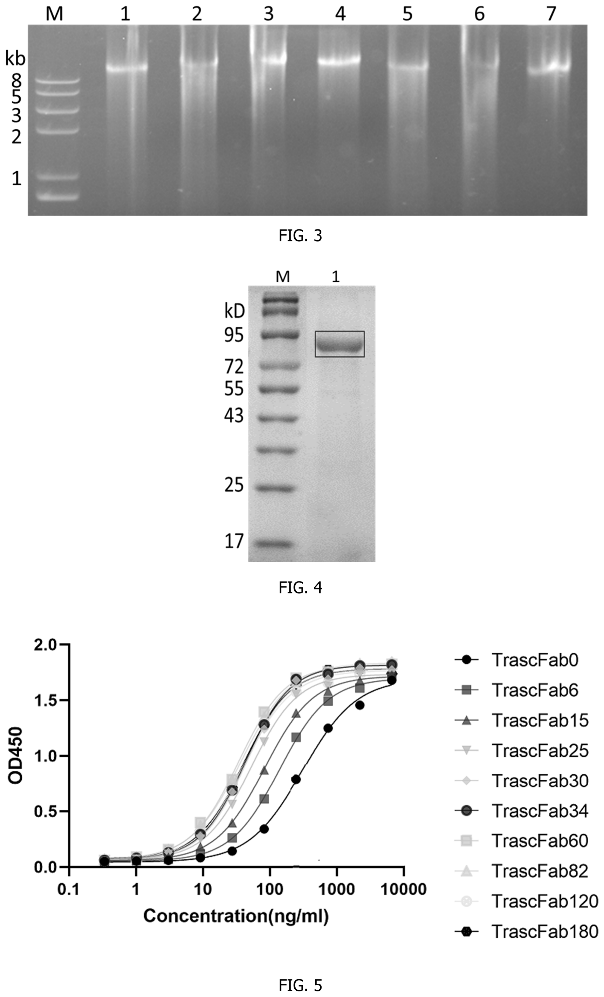

FIG. 3 shows the purification analysis of the AMPI products from Examples 2 and 7 of the present disclosure, in which M represents the marker, and lanes 1 to 7 represent the electrophoresis results of the AMPI products of TrascFab0, TrascFab6, TrascFab34, TrascFab60, TrascFab82, TrascFab120, and scIgG60, respectively.
FIG. 4 shows the analysis of the purified TrascFab60 protein from Example 6 of the present disclosure, in which M represents the marker, and lane 1 represents the purified TrascFab60 protein sample.
FIG. 5 shows the affinity detection results of purified TrascFab proteins with different linker lengths in Example 8 of the present disclosure, including TrascFab0, TrascFab6, TrascFab15, TrascFab25, TrascFab30, TrascFab34, TrascFab60, TrascFab82, TrascFab120, and TrascFab180, in which Concentration indicates the concentration.

### DETAILED DESCRIPTION OF THE PREFERRED EMBODIMENT

The advantages and features of the present disclosure will become more apparent from the following description. However, the examples described herein are merely illustrative and do not limit the scope of the present disclosure. It should be understood by those skilled in the art that various modifications or substitutions may be made to the details and forms of the technical solutions of the present disclosure without departing from the spirit and scope of the present disclosure, but such modifications and substitutions shall fall within the scope of protection of the present disclosure.

### Terminology

As used herein, the term "antibody" refers to a protective protein produced by the body in response to antigen stimulation, which is a type of immunoglobulin capable of specifically binding to antigens. It contains four heterologous polypeptide chains, in which the two chains with higher molecular weight are referred to as heavy chains (H), and the two chains with lower molecular weight are referred to as light chains (L). The two H chains and the two L chains in the same Ig molecule have identical amino acid compositions. The heavy chain has a molecular weight of 50,000 to 75,000 and consists of 450 to 550 amino acid residues, while the light chain has a molecular weight of approximately 25,000 and consists of 214 amino acid residues. Both the two heavy chains and the two light chains of a natural antibody molecule can fold into several spherical domains, each of which performs its corresponding function. The light chain comprises two domains: VL and CL; the heavy chains of IgG, IgA, and IgD comprise four domains: VH, CH1, CH2, and CH3; the heavy chains of IgM and IgE comprise five domains, with an additional CH4 domain.

As used herein, the term "light chain fragment" refers to a structural fragment capable of performing the corresponding function of the light chain, such as a structural fragment comprising the CL domain.

As used herein, the term "heavy chain fragment" refers to a structural fragment capable of performing the corresponding function of the heavy chain, such as a structural fragment comprising the CH1 domain.

As used herein, the term *"in vitro* synthesis system" includes, but is not limited to, an IVTT reaction *(in vitro* transcription and translation reaction). In the present disclosure, the IVTT reaction is preferred. The IVTT reaction, corresponding to an IVTT system, is a process of transcribing and translating DNA into protein *in vitro.* Accordingly, such *in vitro* protein synthesis systems are also referred to as the D2P system, D-to-P system, D_to_P system, or DNA-to-Protein system. The corresponding *in vitro* protein synthesis methods are also referred to as the D2P method, D-to-P method, D_to_P method, or DNA-to-Protein method, which have the same meaning as the expressions *"in vitro* cell-free protein synthesis system", *"in vitro* expression system", *"in vitro* protein synthesis system", *"in vitro* protein synthesis reaction system", and "cell-free protein synthesis system". Other synonymous expressions include protein *in vitro* synthesis system, *in vitro* protein synthesis system, cell-free system, cell-free protein synthesis system, cell-free *in vitro* protein synthesis system, *in vitro* cell-free protein synthesis system, *in vitro* cell-free synthesis system, CFS system (cell-free system), and CFPS system (cell-free protein synthesis system). These also encompass *in vitro* translation system and *in vitro* transcription and translation system (IVTT system). The *in vitro* protein synthesis system is also referred to as a "Protein Factory". The term *"in vitro* protein synthesis reaction" refers to the synthesis of proteins in an *in vitro* cell-free synthesis system, which at least includes the translation process.

A first aspect of the present disclosure provides a single-chain antibody, wherein the single-chain antibody is formed by connecting a light chain or light chain fragment of an antibody to a heavy chain or heavy chain fragment of an antibody via a linker; it is efficiently expressed in one step in an *in vitro* cell-free synthesis system, eliminating the need for separate secretion and expression of two different protein fragments, *i.e.,* the light chain and heavy chain, which is less prone to forming homodimers and exhibits high expression efficiency; and the binding activity of the single-chain antibody to the corresponding antigen is comparable to that of the antibody.

The comparable activity refers to biological comparability, *e.g*., the binding activity of the single-chain antibody to the corresponding antigen reaches 85% to 115% of the activity value of the antibody, preferably 90% to 100%, and more preferably 95% to 100%; the activity value may be any value expressing antibody activity in the art, such as an EC50 value.

Studies have found that single-chain antibodies prepared with linkers of different lengths exhibit varying expression activities. In the present disclosure, linkers containing an appropriate number of amino acid residues are selected through a large number of experiments to achieve both high-efficiency expression and high affinity of the single-chain antibodies.

To balance expression efficiency and specific affinity activity, the linker comprises at least 20 amino acid residues, preferably 25 to 180 amino acid residues, and more preferably 34 to 120 amino acid residues;
for example, the linker comprises 25, 26, 27, 28, 29, 30, 31, 32, or 33 amino acid residues;
for another example, the linker comprises 55, 56, 57, 58, 59, 60, 61, 62, 63, 64, 65, 66, 67, 68, 69, or 70 amino acid residues;
for another example, the linker comprises 115, 116, 117, 118, 119, 120, 121, 122, 123, 124, or 125 amino acid residues.

The linker is named based on the number of amino acid residues contained therein. For example, when the linker comprises 6 amino acid residues, it is named linker6; when the linker comprises 60 amino acid residues, it is named linker60; when the linker comprises 120 amino acid residues, it is named linker120.

Single-chain antibodies prepared with linkers of different lengths exhibit varying expression activities linkers containing an appropriate number of amino acid residues are selected through a large number of experiments to achieve both high-efficiency expression and high affinity of the single-chain antibodies. The amino acids comprised in the linker are selected from serine (S), glycine (G), glutamic acid (E), threonine (T), alanine (A), arginine (R), tyrosine (Y), isoleucine (I), lysine (K), leucine (L), glutamine (Q), histidine (H), phenylalanine (F), valine (V), proline (P), aspartic acid (D), methionine (M), and asparagine (N).

Preferably, the amino acids comprised in the linker are selected from serine (S), glycine (G), glutamic acid (E), threonine (T), and alanine (A).

In a preferred embodiment, the total number of serine (S), glycine (G), glutamic acid (E), threonine (T), and alanine (A) comprised in the linker accounts for 100%, at least 99%, at least 98%, at least 97%, at least 96%, at least 95%, at least 90%, at least 85%, at least 80%, at least 75%, at least 70%, at least 65%, at least 60%, at least 55%, and at least 50% of the total number of amino acids in the linker.

In another preferred embodiment, the total number of serine (S), glycine (G), and glutamic acid (E) comprised in the linker accounts for 100%, at least 99%, at least 98%, at least 97%, at least 96%, at least 95%, at least 90%, at least 85%, at least 80%, at least 75%, at least 70%, at least 65%, at least 60%, at least 55%, and at least 50% of the total number of amino acids in the linker.

In another preferred embodiment, the total number of serine (S), glycine (G), and glutamic acid (E) comprised in the linker accounts for 100%, at least 99%, at least 98%, at least 97%, at least 96%, at least 95%, at least 90%, at least 85%, at least 80%, at least 75%, at least 70%, at least 65%, at least 60%, at least 55%, and at least 50% of the total number of amino acids in the linker.

In a preferred embodiment, the linker is linker25, having the amino acid sequence shown in SEQ ID NO: 11 or a sequence having one or several amino acid substitutions, deletions, or additions relative to the amino acid sequence shown in SEQ ID NO: 11, wherein the number of the amino acid substitutions, deletions, or additions may be 1 to 10, preferably 1 to 5.

SEQ ID NO: 11: GGGGSGGGGSGGGGSGGGGSGGGGS
In a preferred embodiment, the linker is linker30, having the amino acid sequence shown in SEQ ID NO: 14 or a sequence having one or several amino acid substitutions, deletions, or additions relative to the amino acid sequence shown in SEQ ID NO: 14, wherein the number of the amino acid substitutions, deletions, or additions may be 1 to 10, preferably 1 to 5.

SEQ ID NO: 14: SGGGSGGGSEGGGSEGGGSEGGGSEGGGSG
In a preferred embodiment, the linker is linker34, having the amino acid sequence shown in SEQ ID NO: 15 or a sequence having one or several amino acid substitutions, deletions, or additions relative to the amino acid sequence shown in SEQ ID NO: 15, wherein the number of the amino acid substitutions, deletions, or additions may be 1 to 10, preferably 1 to 5.

SEQ ID NO: 15: SGGGSGGGSEGGGSEGGGSEGGGSEGGGSGGGSG
In a preferred embodiment, the linker is linker34A, having the amino acid sequence shown in SEQ ID NO: 16 or a sequence having one or several amino acid substitutions, deletions, or additions relative to the amino acid sequence shown in SEQ ID NO: 16, wherein the number of the amino acid substitutions, deletions, or additions may be 1 to 10, preferably 1 to 5.

SEQ ID NO: 16: RRYEERRYEERRYEERRYEERRYEERRYEERRYE
In a preferred embodiment, the linker is linker34B, having the amino acid sequence shown in SEQ ID NO: 17 or a sequence having one or several amino acid substitutions, deletions, or additions relative to the amino acid sequence shown in SEQ ID NO: 17, wherein the number of the amino acid substitutions, deletions, or additions may be 1 to 10, preferably 1 to 5.

SEQ ID NO: 17: YYEYYYYEYYYYEYYYYEYYYYEYYYYEYYEYYE
In a preferred embodiment, the linker is linker34C, having the amino acid sequence shown in SEQ ID NO: 18 or a sequence having one or several amino acid substitutions, deletions, or additions relative to the amino acid sequence shown in SEQ ID NO: 18, wherein the number of the amino acid substitutions, deletions, or additions may be 1 to 10, preferably 1 to 5.

SEQ ID NO: 18: IKYLEFISEAIIHVLHSRHPGDFGADAQGAMNKA
In a preferred embodiment, the linker is linker60, having the amino acid sequence shown in SEQ ID NO: 19 or a sequence having one or several amino acid substitutions, deletions, or additions relative to the amino acid sequence shown in SEQ ID NO: 19, wherein the number of the amino acid substitutions, deletions, or additions may be 1 to 50, preferably 1 to 20.

SEQ ID NO: 19:
GGSSGSGSGSTGTSSSGTGTSAGTTGTSASTSGSGSGGGGGSGGGGSAGGTATAGASSGS
In a preferred embodiment, the linker is linker60A, having the amino acid sequence shown in SEQ ID NO: 20 or a sequence having one or several amino acid substitutions, deletions, or additions relative to the amino acid sequence shown in SEQ ID NO: 20, wherein the number of the amino acid substitutions, deletions, or additions may be 1 to 50, preferably 1 to 20.

SEQ ID NO: 20:
LAQSHATKHKIPIKYLEFISEAIIHVLHSRHPGDFGADAQGAMNKALELFRKDIAAKYKE
In a preferred embodiment, the linker is linker82, having the amino acid sequence shown in SEQ ID NO: 21 or a sequence having one or several amino acid substitutions, deletions, or additions relative to the amino acid sequence shown in SEQ ID NO: 21, wherein the number of the amino acid substitutions, deletions, or additions may be 1 to 50, preferably 1 to 20.

SEQ ID NO: 21:

GGSSGSGSGSTGTSSSGTGTSAGTTGTSASTSGSGSGGGGGSGGGGSAGGTATAAGSTGGAGST
GGAGSTGGAGTGSGAGS

In a preferred embodiment, the linker is linker120, having the amino acid sequence shown in SEQ ID NO: 22 or a sequence having one or several amino acid substitutions, deletions, or additions relative to the amino acid sequence shown in SEQ ID NO: 22, wherein the number of the amino acid substitutions, deletions, or additions may be 1 to 50, preferably 1 to 20.

SEQ ID NO: 22:

GGSSGSGSGSTGTSSSGTGTSAGTTGTSASTSGSGSGGGGGSGGGGSAGGTATGGSSGGSGGSS

GSGSGSTGTSSSGTGTSAGTTGTSASTSGSGSGGGGGSGGGGSAGGTATAGASSGS

In a preferred embodiment, the linker is linker180, having the amino acid sequence shown in SEQ ID NO: 23 or a sequence having one or several amino acid substitutions, deletions, or additions relative to the amino acid sequence shown in SEQ ID NO: 23, wherein the number of the amino acid substitutions, deletions, or additions may be 1 to 50, preferably 1 to 20.

SEQ ID NO: 23:

GGSSGSGSGSTGTSSSGTGTSAGTTGTSASTSGSGSGGGGGSGGGGSAGGTATAGASSGSGGSS
GSGSGSTGTSSSGTGTSAGTTGTSASTSGSGSGGGGGSGGGGSAGGTATAGASSGSGGSSGSGSGST
GTSSSGTGTSAGTTGTSASTSGSGSGGGGGSGGGGSAGGTATAGASSGS

In a preferred embodiment, the linker is Linker36A, having the amino acid sequence shown in SEQ ID NO: 24 or a sequence having one or several amino acid substitutions, deletions, or additions relative to the amino acid sequence shown in SEQ ID NO: 24, wherein the number of the amino acid substitutions, deletions, or additions may be 1 to 50, preferably 1 to 20.

SEQ ID NO: 24: GSGVPSRFSGSGSGTDFTLTISSLQPEDFATYYSGS
In a preferred embodiment, the linker is Linker36B, having the amino acid sequence shown in SEQ ID NO: 25 or a sequence having one or several amino acid substitutions, deletions, or additions relative to the amino acid sequence shown in SEQ ID NO: 25, wherein the number of the amino acid substitutions, deletions, or additions may be 1 to 50, preferably 1 to 20.

SEQ ID NO: 25: GSGEVQLVESGGGLVQPGGSLRLSSAASGFNIKGSG
In a preferred embodiment, the linker is Linker36C, having the amino acid sequence shown in SEQ ID NO: 26 or a sequence having one or several amino acid substitutions, deletions, or additions relative to the amino acid sequence shown in SEQ ID NO: 26, wherein the number of the amino acid substitutions, deletions, or additions may be 1 to 50, preferably 1 to 20.

SEQ ID NO: 26: GAGAGSGAGAGSGAGAGSGAGAGSGAGAGSGAGAGS
The antibody according to the present disclosure is selected from IgM, IgG, IgA, IgD, and/or IgE antibodies;
further preferably, the IgG antibody is selected from IgG1, IgG2, IgG3 and/or IgG4;
in a preferred embodiment, the antibody is selected from an IgG1 antibody; preferably, the IgG1 antibody is Trastuzumab, Bevacizumab, Daratumumab, Omalizumab, Cetuximab, Adalimumab, Ustekinumab, Ocrelizumab, Infliximab, Envafolimab, Atlizumab, and/or Atezolizumab;
the IgG2 antibody is Denosumab, Erenumab, Tremelimumab, or Evocumab;
the IgG4 antibody is Tislelizumab, Dupilumab, Rituximab, Pembrolizumab, Nivolumab, or Palivizumab.

The antibody light chain is selected from a κ chain and/or a λ chain; the antibody light chain fragment is selected from a κ chain fragment and a λ chain fragment; the heavy chain is selected from a µ chain, a γ chain, an α chain, a δ chain, and/or an ε chain; the heavy chain fragment is selected from a µ chain fragment, a γ chain fragment, an α chain fragment, a δ chain fragment, and/or an ε chain fragment.

In a preferred embodiment, the antibody light chain is derived from Trastuzumab light chain TraLC, and the antibody heavy chain is derived from Trastuzumab heavy chain TraHC; in another preferred embodiment, the antibody light chain is derived from Trastuzumab light chain TraLC, and the antibody heavy chain fragment is a Trastuzumab heavy chain fragment TraFd.

Furthermore, the Trastuzumab light chain TraLC comprises the amino acid sequence shown in SEQ ID NO: 4, or a sequence having one or more amino acid substitutions, deletions, or additions relative to the polypeptide shown in the amino acid sequence shown in SEQ ID NO: 4, wherein the number of the amino acid substitutions, deletions, or additions may be 1 to 50, preferably 1 to 10, such as 1, 2, 3, 4, 5, 6, 7, 8, 9, or 10.

SEQ ID NO: 4:

DIQMTQSPSSLSASVGDRVTITCRASQDVNTAVAWYQQKPGKAPKLLIYSASFLYSGVPSRFSGSR
SGTDFTLTISSLQPEDFATYYCQQHYTTPPTFGQGTKVEIKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPV
TKSFNRGEC

Preferably, the Trastuzumab light chain TraLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 4.

Furthermore, the Trastuzumab heavy chain fragment TraFd comprises the amino acid sequence shown in SEQ ID NO: 5, or a sequence having one or more amino acid substitutions, deletions, or additions relative to the polypeptide shown in SEQ ID NO: 5, wherein the number of the amino acid substitutions, deletions, or additions may be 1 to 100, preferably 1 to 20, such as 1, 2, 3, 4, 5, 6, 7, 8, 9, or 10.

SEQ ID NO: 5:

EVQLVESGGGLVQPGGSLRLSCAASGFNIKDTYIHWVRQAPGKGLEWVARIYPTNGYTRYADSVK
GRFTISADTSKNTAYLQMNSLRAEDTAVYYCSRWGGDGFYAMDYWGQGTLVTVSSASTKGPSVFPLAP
SSKSTSGGTAALGCLVKDYFPEPVTVSWNSGALTSGVHTFPAVLQSSGLYSLSSVVTVPSSSLGTQTYIC
NVNHKPSNTKVDKKVEPKSCDK

Preferably, the Trastuzumab heavy chain fragment TraFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 5.

Furthermore, the Trastuzumab heavy chain TraHC comprises the amino acid sequence shown in SEQ ID NO: 6, or a sequence having one or more amino acid substitutions, deletions, or additions relative to the polypeptide shown in SEQ ID NO: 6, wherein the number of the amino acid substitutions, deletions, or additions may be 1 to 100, preferably 1 to 20, such as 1, 2, 3, 4, 5, 6, 7, 8, 9, or 10.

SEQ ID NO: 6:

EVQLVESGGGLVQPGGSLRLSCAASGFNIKDTYIHWVRQAPGKGLEWVARIYPTNGYTRYADSVK
GRFTISADTSKNTAYLQMNSLRAEDTAVYYCSRWGGDGFYAMDYWGQGTLVTVSSASTKGPSVFPLAP
SSKSTSGGTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

Preferably, the Trastuzumab heavy chain fragment TraHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 6.

In a preferred embodiment, the antibody light chain is derived from Tislelizumab light chain TisLC or a fragment thereof, and the antibody heavy chain is derived from Tislelizumab heavy chain TisHC or a fragment TisFd thereof.

Furthermore, the Tislelizumab light chain TisLC comprises the amino acid sequence shown in SEQ ID NO: 27, or a sequence having one or more amino acid substitutions, deletions, or additions relative to the polypeptide shown in the amino acid sequence shown in SEQ ID NO: 27, wherein the number of the amino acid substitutions, deletions, or additions may be 1 to 50, preferably 1 to 10, such as 1, 2, 3, 4, 5, 6, 7, 8, 9, or 10.

SEQ ID NO: 27:

DIVMTQSPDSLAVSLGERATINCKSSESVSNDVAWYQQKPGQPPKLLINYAFHRFTGVPDRFSGSG
YGTDFTLTISSLQAEDVAVYYCHQAYSSPYTFGQGTKLEIKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPV
TKSFNRGEC

Preferably, the Tislelizumab light chain TisLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 27.

Furthermore, the Tislelizumab heavy chain TisHC comprises the amino acid sequence shown in SEQ ID NO: 28, or a sequence having one or more amino acid substitutions, deletions, or additions relative to the polypeptide shown in SEQ ID NO: 28, wherein the number of the amino acid substitutions, deletions, or additions may be 1 to 100, preferably 1 to 20, such as 1, 2, 3, 4, 5, 6, 7, 8, 9, or 10.

SEQ ID NO: 28:

QVQLQESGPGLVKPSETLSLTCTVSGFSLTSYGVHWIRQPPGKGLEWIGVIYADGSTNYNPSLKSR
VTISKDTSKNQVSLKLSSVTAADTAVYYCARAYGNYWYIDVWGQGTTVTVSSASTKGPSVFPLAPCSRS
TSESTAALGCLVKDYFPEPVTVSWNSGALTSGVHTFPAVLQSSGLYSLSSVVTVPSSSLGTKTYTCNVDH
KPSNTKVDKRVESKYGPPCPPCPAPPVAGGPSVFLFPPKPKDTLMISRTPEVTCVVVAVSQEDPEVQFN
WYVDGVEVHNAKTKPREEQFNSTYRVVSVLTVVHQDWLNGKEYKCKVSNKGLPSSIEKTISKAKGQPR
EPQVYTLPPSQEEMTKNQVSLTCLVKGFYPSDIAVEWESNGQPENNYKTTPPVLDSDGSFFLYSKLTVD
KSRWQEGNVFSCSVMHEALHNHYTQKSLSLSLGKTGGAGTGSGA

Preferably, the Tislelizumab heavy chain TisHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 28.

Furthermore, the Tislelizumab heavy chain fragment TisFd comprises the amino acid sequence shown in SEQ ID NO: 29, or a sequence having one or more amino acid substitutions, deletions, or additions relative to the polypeptide shown in SEQ ID NO: 29, wherein the number of the amino acid substitutions, deletions, or additions may be 1 to 100, preferably 1 to 20, such as 1, 2, 3, 4, 5, 6, 7, 8, 9, or 10.

SEQ ID NO: 29:

QVQLQESGPGLVKPSETLSLTCTVSGFSLTSYGVHWIRQPPGKGLEWIGVIYADGSTNYNPSLKSR
VTISKDTSKNQVSLKLSSVTAADTAVYYCARAYGNYWYIDVWGQGTTVTVSSASTKGPSVFPLAPCSRS
TSESTAALGCLVKDYFPEPVTVSWNSGALTSGVHTFPAVLQSSGLYSLSSVVTVPSSSLGTKTYTCNVDH
KPSNTKVDKRVESK

Preferably, the Trastuzumab heavy chain fragment TraFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 5.

In a preferred embodiment, the antibody light chain is derived from Dupilumab light chain DupLC or a fragment thereof, and the antibody heavy chain is derived from Dupilumab heavy chain DupHC or a fragment DupFd thereof.

Furthermore, the Dupilumab light chain DupLC comprises the amino acid sequence shown in SEQ ID NO: 30, or a sequence having one or more amino acid substitutions, deletions, or additions relative to the polypeptide shown in the amino acid sequence shown in SEQ ID NO: 30, wherein the number of the amino acid substitutions, deletions, or additions may be 1 to 50, preferably 1 to 10, such as 1, 2, 3, 4, 5, 6, 7, 8, 9, or 10.

SEQ ID NO: 30:

DIVMTQSPLSLPVTPGEPASISCRSSQSLLYSIGYNYLDWYLQKSGQSPQLLIYLGSNRASGVPDRF
SGSGSGTDFTLKISRVEAEDVGFYYCMQALQTPYTFGQGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC

Preferably, the Dupilumab light chain DupLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 30.

Furthermore, the Dupilumab heavy chain DupHC comprises the amino acid sequence shown in SEQ ID NO: 31, or a sequence having one or more amino acid substitutions, deletions, or additions relative to the polypeptide shown in SEQ ID NO: 31, wherein the number of the amino acid substitutions, deletions, or additions may be 1 to 100, preferably 1 to 20, such as 1, 2, 3, 4, 5, 6, 7, 8, 9, or 10.

SEQ ID NO: 31:

EVQLVESGGGLEQPGGSLRLSCAGSGFTFRDYAMTWVRQAPGKGLEWVSSISGSGGNTYYADSV
KGRFTISRDNSKNTLYLQMNSLRAEDTAVYYCAKDRLSITIRPRYYGLDVWGQGTTVTVSSASTKGPSV
FPLAPCSRSTSESTAALGCLVKDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKDTLMISRTPEVTCVVVDVSQE
DPEVQFNWYVDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPPVLDSDGSFFL
YSRLTVDKSRWQEGNVFSCSVMHEALHNHYTQKSLSLSLG

Preferably, the Dupilumab heavy chain DupHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 31.

Furthermore, the Dupilumab heavy chain fragment DupFd comprises the amino acid sequence shown in SEQ ID NO: 32, or a sequence having one or more amino acid substitutions, deletions, or additions relative to the polypeptide shown in SEQ ID NO: 32, wherein the number of the amino acid substitutions, deletions, or additions may be 1 to 100, preferably 1 to 20, such as 1, 2, 3, 4, 5, 6, 7, 8, 9, or 10.

SEQ ID NO: 32:

EVQLVESGGGLEQPGGSLRLSCAGSGFTFRDYAMTWVRQAPGKGLEWVSSISGSGGNTYYADSV
KGRFTISRDNSKNTLYLQMNSLRAEDTAVYYCAKDRLSITIRPRYYGLDVWGQGTTVTVSSASTKGPSV
FPLAPCSRSTSESTAALGCLVKDYFPEPVTVSWNSGALTSGVHTFPAVLQSSGLYSLSSVVTVPSSSLGTK
TYTCNVDHKPSNTKVDKRVESKYG

Preferably, the Dupilumab heavy chain fragment DupFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 32.

In a preferred embodiment, the antibody light chain is derived from Adalimumab light chain AdaLC or a fragment thereof, and the antibody heavy chain is derived from Adalimumab heavy chain AdaHC or a fragment AdaFd thereof.

Furthermore, the Adalimumab light chain AdaLC comprises the amino acid sequence shown in SEQ ID NO: 33, or a sequence having one or more amino acid substitutions, deletions, or additions relative to the polypeptide shown in the amino acid sequence shown in SEQ ID NO: 33, wherein the number of the amino acid substitutions, deletions, or additions may be 1 to 50, preferably 1 to 10, such as 1, 2, 3, 4, 5, 6, 7, 8, 9, or 10.

SEQ ID NO: 33:

DIQMTQSPSSLSASVGDRVTITCRASQGIRNYLAWYQQKPGKAPKLLIYAASTLQSGVPSRFSGSG
SGTDFTLTISSLQPEDVATYYCQRYNRAPYTFGQGTKVEIKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPV
TKSFNRGEC

Preferably, the Adalimumab light chain AdaLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 33.

Furthermore, the Adalimumab heavy chain AdaHC comprises the amino acid sequence shown in SEQ ID NO: 34, or a sequence having one or more amino acid substitutions, deletions, or additions relative to the polypeptide shown in SEQ ID NO: 34, wherein the number of the amino acid substitutions, deletions, or additions may be 1 to 100, preferably 1 to 20, such as 1, 2, 3, 4, 5, 6, 7, 8, 9, or 10.

EVQLVESGGGLVQPGRSLRLSCAASGFTFDDYAMHWVRQAPGKGLEWVSAITWNSGHIDYADSV
EGRFTISRDNAKNSLYLQMNSLRAEDTAVYYCAKVSYLSTASSLDYWGQGTLVTVSSASTKGPSVFPLA
PSSKSTSGGTAALGCLVKDYFPEPVTVSWNSGALTSGVHTFPAVLQSSGLYSLSSVVTVPSSSLGTQTYI
CNVNHKPSNTKVDKKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGKTGGAGTGSGA

Preferably, the Adalimumab heavy chain AdaHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 34.

Furthermore, the Adalimumab heavy chain fragment AdaFd comprises the amino acid sequence shown in SEQ ID NO: 35, or a sequence having one or more amino acid substitutions, deletions, or additions relative to the polypeptide shown in SEQ ID NO: 35, wherein the number of the amino acid substitutions, deletions, or additions may be 1 to 100, preferably 1 to 20, such as 1, 2, 3, 4, 5, 6, 7, 8, 9, or 10.

SEQ ID NO: 35:

EVQLVESGGGLVQPGRSLRLSCAASGFTFDDYAMHWVRQAPGKGLEWVSAITWNSGHIDYADSV
EGRFTISRDNAKNSLYLQMNSLRAEDTAVYYCAKVSYLSTASSLDYWGQGTLVTVSSASTKGPSVFPLA
PSSKSTSGGTAALGCLVKDYFPEPVTVSWNSGALTSGVHTFPAVLQSSGLYSLSSVVTVPSSSLGTQTYI
CNVNHKPSNTKVDKKVEPKSCDK

Preferably, the Adalimumab heavy chain fragment AdaFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 35.

In a preferred embodiment, the antibody light chain is derived from Bevacizumab light chain BevLC or a fragment thereof, and the antibody heavy chain is derived from Bevacizumab heavy chain BevHC or a fragment BevFd thereof.

Furthermore, the Bevacizumab light chain BevLC comprises the amino acid sequence shown in SEQ ID NO: 36, or a sequence having one or more amino acid substitutions, deletions, or additions relative to the polypeptide shown in the amino acid sequence shown in SEQ ID NO: 36, wherein the number of the amino acid substitutions, deletions, or additions may be 1 to 50, preferably 1 to 10, such as 1, 2, 3, 4, 5, 6, 7, 8, 9, or 10.

SEQ ID NO: 36:

DIQMTQSPSSLSASVGDRVTITCSASQDISNYLNWYQQKPGKAPKVLIYFTSSLHSGVPSRFSGSG
SGTDFTLTISSLQPEDFATYYCQQYSTVPWTFGQGTKVEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC

Preferably, the Bevacizumab light chain BevLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 36.

Furthermore, the Bevacizumab heavy chain BevHC comprises the amino acid sequence shown in SEQ ID NO: 37, or a sequence having one or more amino acid substitutions, deletions, or additions relative to the polypeptide shown in SEQ ID NO: 37, wherein the number of the amino acid substitutions, deletions, or additions may be 1 to 100, preferably 1 to 20, such as 1, 2, 3, 4, 5, 6, 7, 8, 9, or 10.

SEQ ID NO: 37:

EVQLVESGGGLVQPGGSLRLSCAASGYTFTNYGMNWVRQAPGKGLEWVGWINTYTGEPTYAADF
KRRFTFSLDTSKSTAYLQMNSLRAEDTAVYYCAKYPHYYGSSHWYFDVWGQGTLVTVSSASTKGPSVF
PLAPSSKSTSGGTAALGCLVKDYFPEPVTVSWNSGALTSGVHTFPAVLQSSGLYSLSSVVTVPSSSLGTQ
TYICNVNHKPSNTKVDKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPAPIEK
TISKAKGQPREPQVYTLPPSREEMTKNQVSLTCLVKGFYPSDIAVEWESNGQPENNYKTTPPVLDSDGS
FFLYSKLTVDKSRWQQGNVFSCSVMHEALHNHYTQKSLSLSPGKTGGAGTGSGA

Preferably, the Bevacizumab heavy chain BevHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 37.

Furthermore, the Bevacizumab heavy chain fragment BevFd comprises the amino acid sequence shown in SEQ ID NO: 38, or a sequence having one or more amino acid substitutions, deletions, or additions relative to the polypeptide shown in SEQ ID NO: 38, wherein the number of the amino acid substitutions, deletions, or additions may be 1 to 100, preferably 1 to 20, such as 1, 2, 3, 4, 5, 6, 7, 8, 9, or 10.

SEQ ID NO: 38:

EVQLVESGGGLVQPGGSLRLSCAASGYTFTNYGMNWVRQAPGKGLEWVGWINTYTGEPTYAADF
KRRFTFSLDTSKSTAYLQMNSLRAEDTAVYYCAKYPHYYGSSHWYFDVWGQGTLVTVSSASTKGPSVF
PLAPSSKSTSGGTAALGCLVKDYFPEPVTVSWNSGALTSGVHTFPAVLQSSGLYSLSSVVTVPSSSLGTQ
TYICNVNHKPSNTKVDKKVEPKSCDK

Preferably, the Bevacizumab heavy chain fragment BevFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 38.

In a preferred embodiment, the antibody light chain is derived from Daratumumab light chain DarLC or a fragment thereof, and the antibody heavy chain is derived from Daratumumab heavy chain DarHC or a fragment DarFd thereof.

Furthermore, the Daratumumab light chain DarLC comprises the amino acid sequence shown in SEQ ID NO: 39, or a sequence having one or more amino acid substitutions, deletions, or additions relative to the polypeptide shown in the amino acid sequence shown in SEQ ID NO: 39, wherein the number of the amino acid substitutions, deletions, or additions may be 1 to 50, preferably 1 to 10, such as 1, 2, 3, 4, 5, 6, 7, 8, 9, or 10.

SEQ ID NO: 39:

EIVLTQSPATLSLSPGERATLSCRASQSVSSYLAWYQQKPGQAPRLLIYDASNRATGIPARFSGSGS
GTDFTLTISSLEPEDFAVYYCQQRSNWPPTFGQGTKVEIKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPV
TKSFNRGEC

Preferably, the Daratumumab light chain DarLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 39.

Furthermore, the Daratumumab heavy chain DarHC comprises the amino acid sequence shown in SEQ ID NO: 40, or a sequence having one or more amino acid substitutions, deletions, or additions relative to the polypeptide shown in SEQ ID NO: 40, wherein the number of the amino acid substitutions, deletions, or additions may be 1 to 100, preferably 1 to 20, such as 1, 2, 3, 4, 5, 6, 7, 8, 9, or 10.

SEQ ID NO: 40:

EVQLLESGGGLVQPGGSLRLSCAVSGFTFNSFAMSWVRQAPGKGLEWVSAISGSGGGTYYADSVK
GRFTISRDNSKNTLYLQMNSLRAEDTAVYFCAKDKILWFGEPVFDYWGQGTLVTVSSASTKGPSVFPLA
PSSKSTSGGTAALGCLVKDYFPEPVTVSWNSGALTSGVHTFPAVLQSSGLYSLSSVVTVPSSSLGTQTYI
CNVNHKPSNTKVDKRVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTPPVLDSDGSFF

LYSKLTVDKSRWQQGNVFSCSVMHEALHNHYTQKSLSLSPGK

Preferably, the Daratumumab heavy chain DarHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 40.

Furthermore, the Daratumumab heavy chain fragment DarFd comprises the amino acid sequence shown in SEQ ID NO: 41, or a sequence having one or more amino acid substitutions, deletions, or additions relative to the polypeptide shown in SEQ ID NO: 41, wherein the number of the amino acid substitutions, deletions, or additions may be 1 to 100, preferably 1 to 20, such as 1, 2, 3, 4, 5, 6, 7, 8, 9, or 10.

SEQ ID NO: 41:

EVQLLESGGGLVQPGGSLRLSCAVSGFTFNSFAMSWVRQAPGKGLEWVSAISGSGGGTYYADSVK
GRFTISRDNSKNTLYLQMNSLRAEDTAVYFCAKDKILWFGEPVFDYWGQGTLVTVSSASTKGPSVFPLA
PSSKSTSGGTAALGCLVKDYFPEPVTVSWNSGALTSGVHTFPAVLQSSGLYSLSSVVTVPSSSLGTQTYI
CNVNHKPSNTKVDKRVEPKSCDK

Preferably, the Daratumumab heavy chain fragment DarFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 41.

In a preferred embodiment, the antibody light chain is derived from Omalizumab light chain OmaLC or a fragment thereof, and the antibody heavy chain is derived from Omalizumab heavy chain OmaHC or a fragment OmaFd thereof.

Furthermore, the Omalizumab light chain OmaLC comprises the amino acid sequence shown in SEQ ID NO: 42, or a sequence having one or more amino acid substitutions, deletions, or additions relative to the polypeptide shown in the amino acid sequence shown in SEQ ID NO: 42, wherein the number of the amino acid substitutions, deletions, or additions may be 1 to 50, preferably 1 to 10, such as 1, 2, 3, 4, 5, 6, 7, 8, 9, or 10.

SEQ ID NO: 42:

DIQLTQSPSSLSASVGDRVTITCRASQSVDYDGDSYMNWYQQKPGKAPKLLIYAASYLESGVPSRF

SGSGSGTDFTLTISSLQPEDFATYYCQQSHEDPYTFGQGTKVEIKRTVAAPSVFIFPPSDEQLKSGTASV

VCLLNNFYPREAKVQWKVDNALQSGNSQESVTEQDSKDSTYSLSSTLTLSKADYEKHKVYACEVTHQG

LSSPVTKSFNR

Preferably, the Omalizumab light chain OmaLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 42.

Furthermore, the Omalizumab heavy chain OmaHC comprises the amino acid sequence shown in SEQ ID NO: 43, or a sequence having one or more amino acid substitutions, deletions, or additions relative to the polypeptide shown in SEQ ID NO: 43, wherein the number of the amino acid substitutions, deletions, or additions may be 1 to 100, preferably 1 to 20, such as 1, 2, 3, 4, 5, 6, 7, 8, 9, or 10.

SEQ ID NO: 43:

EVQLVESGGGLVQPGGSLRLSCAVSGYSITSGYSWNWIRQAPGKGLEWVASITYDGSTNYADSVK

GRFTISRDDSKNTFYLQMNSLRAEDTAVYYCARGSHYFGHWHFAVWGQGTLVTVSSGPSVFPLAPSSK

STSGGTAALGCLVKDYFPEPVTVSWNSGALTSGVHTFPAVLQSSGLYSLSSVVTVPSSSLGTQTYICNVN

HKPSNTKVDKKAEPKSCDKTHTCPPCPAPELLGGPSVFLFPPKPKDTLMISRTPEVTCVVVDVSHEDPEV

KFNWYVDGVEVHNAKTKPREEQYNSTYRVVSVLTVLHQDWLNGKEYKCKVSNKALPAPIEKTISKAKG

QPREPQVYTLPPSRDELTKNQVSLTCLVKGFYPSDIAVEWESNGQPENNYKTTPPVLDSDGSFFLYSKLT

VDKSRWQQGNVFSCSVMHEALHNHYTQKSLSLSPGK

Preferably, the Omalizumab heavy chain OmaHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 43.

Furthermore, the Omalizumab heavy chain fragment OmaFd comprises the amino acid sequence shown in SEQ ID NO: 44, or a sequence having one or more amino acid substitutions, deletions, or additions relative to the polypeptide shown in SEQ ID NO: 44, wherein the number of the amino acid substitutions, deletions, or additions may be 1 to 100, preferably 1 to 20, such as 1, 2, 3, 4, 5, 6, 7, 8, 9, or 10.

SEQ ID NO: 44:

EVQLVESGGGLVQPGGSLRLSCAVSGYSITSGYSWNWIRQAPGKGLEWVASITYDGSTNYADSVK
GRFTISRDDSKNTFYLQMNSLRAEDTAVYYCARGSHYFGHWHFAVWGQGTLVTVSSGPSVFPLAPSSK
STSGGTAALGCLVKDYFPEPVTVSWNSGALTSGVHTFPAVLQSSGLYSLSSVVTVPSSSLGTQTYICNVN
HKPSNTKVDKKAEPKSCDK

Preferably, the Omalizumab heavy chain fragment OmaFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 44.

In a preferred embodiment, the antibody light chain is derived from Cetuximab light chain CetLC or a fragment thereof, and the antibody heavy chain is derived from Cetuximab heavy chain CetHC or a fragment CetFd thereof.

Furthermore, the Cetuximab light chain CetLC comprises the amino acid sequence shown in SEQ ID NO: 45, or a sequence having one or more amino acid substitutions, deletions, or additions relative to the polypeptide shown in the amino acid sequence shown in SEQ ID NO: 45, wherein the number of the amino acid substitutions, deletions, or additions may be 1 to 50, preferably 1 to 10, such as 1, 2, 3, 4, 5, 6, 7, 8, 9, or 10.

SEQ ID NO: 45:

DVQVTQSPSSLSASVGDRVTITCRSSQSLANSYGNTFLSWYLHKPGKAPQLLIYGISNRFSGVPDR
FSGSGSGTDFTLTISSLQPEDFATYYCLQGTHQPYTFGQGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACEVTHQ
GLSSPVTKSFNRGEC

Preferably, the Cetuximab light chain CetLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 45.

Furthermore, the Cetuximab heavy chain CetHC comprises the amino acid sequence shown in SEQ ID NO: 46, or a sequence having one or more amino acid substitutions, deletions, or additions relative to the polypeptide shown in SEQ ID NO: 46, wherein the number of the amino acid substitutions, deletions, or additions may be 1 to 100, preferably 1 to 20, such as 1, 2, 3, 4, 5, 6, 7, 8, 9, or 10.

SEQ ID NO: 46:

EVQLVQSGAEVKKPGASVKVSCKASGYRFTNYWIHWVRQAPGQGLEWIGGINPGNNYATYRRKF
QGRVTMTADTSTSTVYMELSSLRSEDTAVYYCTREGYGNYGAWFAYWGQGTLVTVSSASTKGPSVFPL
APCSRSTSESTAALGCLVKDYFPEPVTVSWNSGALTSGVHTFPAVLQSSGLYSLSSVVTVPSSSLGTKTY
TCNVDHKPSNTKVDKRVESKYGPPCPPCPAPEFLGGPSVFLFPPKPKDTLMISRTPEVTCVVVDVSQEDP
EVQFNWYVDGVEVHNAKTKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPENNYKTTPPVLDSDGSFFLYS
RLTVDKSRWQEGNVFSCSVMHEALHNHYTQKSLSLSLGK

Preferably, the Cetuximab heavy chain CetHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 46.

Furthermore, the Cetuximab heavy chain fragment CetFd comprises the amino acid sequence shown in SEQ ID NO: 47, or a sequence having one or more amino acid substitutions, deletions, or additions relative to the polypeptide shown in SEQ ID NO: 47, wherein the number of the amino acid substitutions, deletions, or additions may be 1 to 100, preferably 1 to 20, such as 1, 2, 3, 4, 5, 6, 7, 8, 9, or 10.

SEQ ID NO: 47:

EVQLVQSGAEVKKPGASVKVSCKASGYRFTNYWIHWVRQAPGQGLEWIGGINPGNNYATYRRKF
QGRVTMTADTSTSTVYMELSSLRSEDTAVYYCTREGYGNYGAWFAYWGQGTLVTVSSASTKGPSVFPL
APCSRSTSESTAALGCLVKDYFPEPVTVSWNSGALTSGVHTFPAVLQSSGLYSLSSVVTVPSSSLGTKTY
TCNVDHKPSNTKVDKRVES

Preferably, the Cetuximab heavy chain fragment CetFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 47.

In a preferred embodiment, the antibody light chain is derived from Denosumab light chain DenLC or a fragment thereof, and the antibody heavy chain is derived from Denosumab heavy chain DenHC or a fragment DenFd thereof.

Furthermore, the Denosumab light chain DenLC comprises the amino acid sequence shown in SEQ ID NO: 48, or a sequence having one or more amino acid substitutions, deletions, or additions relative to the polypeptide shown in the amino acid sequence shown in SEQ ID NO: 48, wherein the number of the amino acid substitutions, deletions, or additions may be 1 to 50, preferably 1 to 10, such as 1, 2, 3, 4, 5, 6, 7, 8, 9, or 10.

SEQ ID NO: 48:

EIVLTQSPGTLSLSPGERATLSCRASQSVRGRYLAWYQQKPGQAPRLLIYGASSRATGIPDRFSGS

GSGTDFTLTISRLEPEDFAVFYCQQYGSSPRTFGQGTKVEIKRTVAAPSVFIFPPSDEQLKSGTASVVCLL

NNFYPREAKVQWKVDNALQSGNSQESVTEQDSKDSTYSLSSTLTLSKADYEKHKVYACEVTHQGLSSP

VTKSFNRGEC

Preferably, the Denosumab light chain DenLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 48.

Furthermore, the Denosumab heavy chain DenHC comprises the amino acid sequence shown in SEQ ID NO: 49, or a sequence having one or more amino acid substitutions, deletions, or additions relative to the polypeptide shown in SEQ ID NO: 49, wherein the number of the amino acid substitutions, deletions, or additions may be 1 to 100, preferably 1 to 20, such as 1, 2, 3, 4, 5, 6, 7, 8, 9, or 10.

SEQ ID NO: 49:

EVQLLESGGGLVQPGGSLRLSCAASGFTFSSYAMSWVRQAPGKGLEWVSGITGSGGSTYYADSVK
GRFTISRDNSKNTLYLQMNSLRAEDTAVYYCAKDPGTTVIMSWFDPWGQGTLVTVSSASTKGPSVFPL
APSSKSTSGGTAALGCLVKDYFPEPVTVSWNSGALTSGVHTFPAVLQSSGLYSLSSVVTVPSSSLGTQTY
ICNVNHKPSNTKVDKKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK

Preferably, the Denosumab heavy chain DenHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 49.

Furthermore, the Denosumab heavy chain fragment DenFd comprises the amino acid sequence shown in SEQ ID NO: 50, or a sequence having one or more amino acid substitutions, deletions, or additions relative to the polypeptide shown in SEQ ID NO: 50, wherein the number of the amino acid substitutions, deletions, or additions may be 1 to 100, preferably 1 to 20, such as 1, 2, 3, 4, 5, 6, 7, 8, 9, or 10.

SEQ ID NO: 50:

EVQLLESGGGLVQPGGSLRLSCAASGFTFSSYAMSWVRQAPGKGLEWVSGITGSGGSTYYADSVK
GRFTISRDNSKNTLYLQMNSLRAEDTAVYYCAKDPGTTVIMSWFDPWGQGTLVTVSSASTKGPSVFPL
APSSKSTSGGTAALGCLVKDYFPEPVTVSWNSGALTSGVHTFPAVLQSSGLYSLSSVVTVPSSSLGTQTY
ICNVNHKPSNTKVDKKVEPKSCDK

Preferably, the Denosumab heavy chain fragment DenFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 50.

Furthermore, the single-chain antibody is connected at the N-terminus of the light chain or light chain fragment with a signal peptide SP to improve the expression efficiency and expression level of the single-chain antibody.

Preferably, the SP comprises the amino acid sequence shown in SEQ ID NO: 1 or a sequence having one or more amino acid substitutions, deletions, or additions relative to the sequence shown in SEQ ID NO: 1, wherein the number of the amino acid substitutions, deletions, or additions may be 1 to 10, preferably 1 to 5.

SEQ ID NO: 1: MITETSSPFRSIFSHSGK
Furthermore, the single-chain antibody is connected at the C-terminus of the heavy chain or heavy chain fragment with a fluorescent protein tag. Further preferably, the fluorescent protein tag comprises eGFP, or a protein tag improved based on eGFP, such as mTagBFP2, moxCerulean3, AmCyanl, MiCy, ZsGreen, Clover, mVenus, ZsYellow1, mKO2, TurboRFP, tdTomato, eqFP611, mKate1.3, mNeptune2, miRFP670, mAmetrine, PAmCherry2, and mEos3.2.

Furthermore, the eGFP comprises the amino acid sequence shown in SEQ ID NO: 8, or a sequence having one or more amino acid substitutions, deletions, or additions relative to the amino acid sequence shown in SEQ ID NO: 8, wherein the number of the amino acid substitutions, deletions, or additions may be 1 to 20, preferably 1 to 10, such as 1, 2, 3, 4, 5, 6, 7, 8, 9, or 10.

SEQ ID NO: 8:

VSKGEELFTGVVPILVELDGDVNGHKFSVRGEGEGDATNGKLTLKFICTTGKLPVPWPTLVTTLTYG
VQCFSRYPDHMKQHDFFKSAMPEGYVQERTISFKDDGTYKTRAEVKFEGDTLVNRIELKGIDFKEDGNI
LGHKLEYNFNSHNVYITADKQKNGIKANFKIRHNVEDGSVQLADHYQQNTPIGDGPVLLPDNHYLSTQ
SKLSKDPNEKRDHMVLLEFVTAAGITLGMDELYK

Furthermore, the single-chain antibody is connected at the N-terminus of the light chain or light chain fragment with a tag label. Preferably, the tag label added at the N-terminus of the light chain or light chain fragment is a His tag, wherein the number of histidine residues in the His tag is 3 to 15, preferably 5 to 10. The His tag is named based on the number of histidine residues. For example, when the His tag comprises 8 histidine residues, it is named 8 His; when the His tag comprises 10 histidine residues, it is named 10 His.

Further preferably, the fluorescent protein tag, the tag label, or the signal peptide is connected to the light chain, light chain fragment, heavy chain, or heavy chain fragment of the antibody via a flexible linker; preferably, the flexible linker comprises 1 to 20 amino acid residues.

Examples of flexible linkers may include 5L (SEQ ID NO: 3: GSGGS), 10L (SEQ ID NO: 7: TGGAGTGSGA), *etc.*

In a preferred embodiment, the single-chain antibody is TrascFab, which comprises the structure of formula (I):
TraLC-linker-TraFd (I)
TraLC is the Trastuzumab light chain TraLC, which comprises the amino acid sequence shown in SEQ ID NO: 4, or a sequence having one or more amino acid substitutions, deletions, or additions relative to the polypeptide shown in SEQ ID NO: 4, and has at least 60%, preferably at least 65%, preferably at least 70%, more preferably at least 75%, more preferably at least 80%, more preferably at least 85%, even more preferably at least 90%, even more preferably at least 95%, and most preferably at least 99% homology; preferably, the Trastuzumab light chain TraLC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the amino acid sequence shown in SEQ ID NO: 4;
TraFd is the Trastuzumab heavy chain fragment TraFd, which comprises the amino acid sequence shown in SEQ ID NO: 5, or a sequence having one or more amino acid substitutions, deletions, or additions relative to the amino acid sequence shown in SEQ ID NO: 5, wherein the number of the amino acid substitutions, deletions, or additions may be 1 to 20, preferably 1 to 10, such as 1, 2, 3, 4, 5, 6, 7, 8, 9, or 10; preferably, the TraFd has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the sequence shown in SEQ ID NO: 5;
the linker is linker34, linker60, linker82, linker120, or a sequence having at least 60%, preferably at least 65%, preferably at least 70%, more preferably at least 75%, more preferably at least 80%, more preferably at least 85%, even more preferably at least 90%, even more preferably at least 95%, and most preferably at least 99% homology to any one of the aforementioned sequences.

In a preferred embodiment, the single-chain antibody is scIgG, which comprises the structure of formula (II):

TraLC-linker-TraHC

TraHC is the Trastuzumab heavy chain, which comprises the amino acid sequence shown in SEQ ID NO: 6, or a sequence having one or more amino acid substitutions, deletions, or additions relative to the polypeptide shown in SEQ ID NO: 6, wherein the number of the amino acid substitutions, deletions, or additions may be 1 to 100, preferably 1 to 20, such as 1, 2, 3, 4, 5, 6, 7, 8, 9, or 10; the TraHC has at least 60%, preferably at least 65%, preferably at least 70%, more preferably at least 75%, more preferably at least 80%, more preferably at least 85%, even more preferably at least 90%, even more preferably at least 95%, and most preferably at least 99% homology to the sequence shown in SEQ ID NO: 6.

The single-chain antibody formed by connecting the light chain and heavy chain via a linker in the present disclosure may be synthesized in one step in an *in vitro* cell-free synthesis system. Compared to Fab, it eliminates the need for separate secretion and expression of two different protein fragments, *i.e.,* the Fab light chain and heavy chain, which is less prone to forming homodimers, and exhibits higher expression efficiency and affinity activity.

A second aspect of the present disclosure provides one or more isolated nucleic acids encoding the single-chain antibody provided in the first aspect.

The nucleic acid is a DNA sequence and/or an RNA sequence.

In a feasible embodiment, the nucleic acid is a linear DNA and is a PCR linear fragment.

Furthermore, the PCR linear fragment may be obtained by amplification techniques, which are not particularly limited, including, but not limited to, PCR amplification, isothermal amplification, constant-temperature amplification, and room-temperature amplification. Specifically, the isothermal amplification is preferably constant-temperature amplification. Commercially available DNA amplification systems may be employed, including, but not limited to, those provided by Biocompare, Neta Scientific Inc, ABM, Thermo Fisher Scientific, Expedeon, and Vivantis.

In a feasible embodiment, the nucleic acid is an RNA, which comprises an amino acid sequence encoding a single-chain antibody.

Furthermore, the RNA is prepared using *in vitro* nucleic acid amplification techniques. The *in vitro* nucleic acid amplification techniques that may be employed are not particularly limited, including, but not limited to, polymerase chain reaction (PCR) amplification, isothermal amplification, constant-temperature amplification, and room-temperature amplification. Examples include loop-mediated isothermal amplification (LAMP), strand displacement amplification (SDA), nucleic acid sequence-based amplification (NASBA), rolling circle amplification (RCA), nicking enzyme-mediated isothermal amplification, helicase-dependent amplification (HDA), transcription-dependent amplification, hybrid capture, transcription-mediated amplification (TMA), recombinase-aided amplification (RAA), recombinase polymerase amplification (RPA), *etc.*

A third aspect of the present disclosure provides one or more expression vectors, comprising a nucleic acid encoding the single-chain antibody provided in the first aspect.

The expression vector includes, but is not limited to, a eukaryotic plasmid vector, a eukaryotic viral vector, a prokaryotic plasmid, a specific vector, a shuttle vector, a minichromosome, and various vectors.

Preferably, the expression vector is a eukaryotic plasmid expression vector and a prokaryotic plasmid expression vector.

In a preferred embodiment, the expression vector is a circular DNA, further preferably a plasmid DNA, such as a pET series plasmid and a pGEM series plasmid.

A fourth aspect of the present disclosure provides a host cell, wherein the host cell contains the vector according to the third aspect of the present disclosure, or has the polynucleotide according to the second aspect integrated into its genome, or expresses the recombinant protein according to the first aspect.

In a preferred embodiment, the host cell is selected from a prokaryotic host cell and a eukaryotic host cell.

In another preferred embodiment, the host cell is a prokaryotic cell, such as *E. coli.*

In another preferred embodiment, the host cell is a eukaryotic cell, such as yeast, a progenitor cell, a Chinese hamster ovary cell, an insect cell, a wheat germ cell, and a rabbit reticulocyte.

Preferably, the yeast is one or more selected from the group consisting of *Saccharomyces cerevisiae* and one or more yeasts of the *Kluyveromyces,* in another preferred embodiment, the *Kluyveromyces* is one or more selected from the group consisting of *Kluyveromyces lactis, Kluyveromyces marxianus,* and *Kluyveromyces dobzhanskii.*

A fifth aspect of the present disclosure provides an *in vitro* cell-free protein synthesis system, comprising the nucleic acid molecule provided in the second aspect or the expression vector provided in the third aspect, or a lysate or extract of the host cell according to the fourth aspect of the present disclosure.

Furthermore, the *in vitro* cell-free protein synthesis system includes, but is not limited to, an *E. coli in vitro* protein synthesis system, a bacterial *in vitro* protein synthesis system, a mammalian cell (*e.g.,* HF9, Hela, CHO, HEK293) *in vitro* protein synthesis system, a plant cell *in vitro* protein synthesis system, a yeast cell *in vitro* protein synthesis system, and an insect cell *in vitro* protein synthesis system. It is preferably a yeast *in vitro* protein synthesis system, more preferably a *Kluyveromyces in vitro* protein synthesis system, and most preferably a *Kluyveromyces lactis* (*K. lactis*) or *Kluyveromyces marxianus in vitro protein* synthesis system.

Furthermore, the *in vitro* protein synthesis system provided by the present disclosure includes, but is not limited to, a cell extract, tris(hydroxymethyl)aminomethane, potassium acetate, magnesium acetate, a mixture of nucleoside triphosphates (NTPs), and a mixture of amino acids.

The cell extract is typically used to provide substances such as ribosomes, transfer RNA (tRNA), aminoacyl-tRNA synthetases, initiation factors and elongation factors required for protein synthesis, and termination release factors, and may also endogenously provide other enzyme substances such as polymerases (RNA polymerase and/or DNA polymerase) after strain modification.

The protein components required in the *in vitro* cell-free protein synthesis system (*e.g.,* RNA polymerase) may be provided endogenously or added exogenously. When provided endogenously, reference may be made to the genetic modification methods provided in existing literature including, but not limited to, CN108690139A, CN109423496A, CN106978439A, CN110408635A, CN110551700A, CN110093284A, CN110845622A, CN110938649A, CN111378708A, CN111484998A, *"*Molecular and Cellular Biology, 1990, 10(1): 353-360", and their cited references. Specifically, the methods include, but are not limited to, inserting the coding sequence into an intracellular free plasmid, integrating the coding gene into the cell genome, and a combination thereof. When provided exogenously, the amount may be controlled and adjusted according to the requirements of the system.

In some preferred embodiments, the yeast cell extract is a *Kluyveromyces* cell extract. Specifically, in some preferred embodiments, the *Kluyveromyces* is *Kluyveromyces lactis* (*K. lactis*).

The *in vitro* protein synthesis systems, templates, plasmids, target proteins, *in vitro* protein synthesis reactions (incubation reactions), various preparation methods, various detection methods as technical elements of the present disclosure may also be independently selected from the suitable embodiments or implementation methods disclosed in the following references, including, but not limited to, CN111484998A, CN106978349A, CN108535489A, CN108690139A, CN108949801A, CN108642076A, CN109022478A, CN109423496A, CN109423497A, CN109423509A, CN109837293A, CN109971783A, CN109988801A, CN109971775A, CN110093284A, CN110408635A, CN110408636A, CN110551745A, CN110551700A, CN110551785A, CN110819647A, CN110845622A, CN110938649A, and CN110964736A. Unless conflicting with the purpose of the present disclosure, these references and their cited references are incorporated by reference in their entirety and for all purposes.

A sixth aspect of the present disclosure provides a use of the single-chain antibody of the present disclosure for the diagnosis, prevention, and treatment of a disease or in the manufacture of a substance for the diagnosis, prevention, and treatment of a disease.

Furthermore, the disease is a tumor and/or cancer, and examples of tumors and/or cancers include, but are not limited to, lung cancer, breast cancer, esophageal cancer, gastric cancer, cervical cancer, colon cancer, rhabdomyosarcoma, liposarcoma, osteosarcoma, lymphoma, leukemia, liver cancer, cervical cancer, ovarian cancer, breast cancer, and other diseases.

In a preferred embodiment, the disease is a disease caused by abnormalities in HER2, TNF-α, VEGF, PD-1, IL-4, IL-13, or CD38.

The present disclosure will be further illustrated below with reference to specific examples and accompanying drawings. It should be understood that these examples are provided solely for the purpose of illustrating the present disclosure and are not intended to limit the scope of the present disclosure.

Experimental methods for which specific conditions are not indicated in the following examples are generally performed according to conventional conditions, such as those described in "Sambrook et al., Molecular Cloning: A Laboratory Manual (New York: Cold Spring Harbor Laboratory Press, 1989)" and "Cell-Free Protein Synthesis: Methods and Protocols [M], Edited by Alexander S. Spirin and James R. Swartz, 2008", or according to the conditions recommended by the manufacturer, or according to or with reference to the conditions guided by the specific embodiments described above. Unless otherwise specified, percentages and parts mentioned in the present disclosure refer to weight percentages and weight parts.

### I. Experiments on single-chain antibodies constructed based on Trastuzumab

To verify the present disclosure, single-chain antibodies were synthesized using an *in vitro* synthesis system, including TrascFab0, TrascFab6, TrascFab15, TrascFab25, TrascFab30, TrascFab34, TrascFab60, TrascFab82, TrascFab120, TrascFab180, TrascFab34A, TrascFab34B, TrascFab34C, TrascFab60A, TrascFab36A, TrascFab36B, TrascFab36B, TrascFab36BHL, TrascIgG60A, and TrascIgG60B. The specific plasmid structures are shown in Table 1.

| Table 1 | |
|---|---|
| Protein name | Protein structure |
| TrascFab0 | SP-8His-5L-TraLC-TraFd-EGFP |
| TrascFab6 | SP-8His-5L-TraLC-linker6-TraFd-EGFP |
| TrascFab15 | SP-8His-5L-TraLC-linker15-TraFd-EGFP |
| TrascFab25 | SP-8His-5L-TraLC-linker25-TraFd-EGFP |
| TrascFab30 | SP-8His-5L-TraLC-linker30-TraFd-EGFP |
| TrascFab34 | SP-8His-5L-TraLC-linker34-TraFd-EGFP |
| TrascFab60 | SP-8His-5L-TraLC-linker60-TraFd-EGFP |
| TrascFab82 | SP-8His-5L-TraLC-linker82-TraFd-EGFP |
| TrascFab120 | SP-8His-5L-TraLC-linker120-TraFd-EGFP |
| TrascFab180 | SP-8His-5L-TraLC-linker180-TraFd-EGFP |
| TrascFab34A | SP-8His-5L-TraLC-linker34A-TraFd-EGFP |
| TrascFab34B | SP-8His-5L-TraLC-linker34B-TraFd-EGFP |
| TrascFab34C | SP-8His-5L-TraLC-linker34C-TraFd-EGFP |
| TrascFab60A | SP-8His-5L-TraLC-linker60A-TraFd-EGFP |
| TrascFab36A | SP-8His-5L-TraLC-linker36A-TraFd-EGFP |
| TrascFab36B | SP-8His-5L-TraLC-linker36B-TraFd-EGFP |
| TrascFab36C | SP-8His-5L-TraLC-linker36C-TraFd-EGFP |
| TrascFabHL | SP-8His-5L-TraFd-linker36C-TraLC-EGFP |
| Tra2Fab | SP-8His-TraLC-linker36B-TraFd-linker20A-TraLC-linker36B-TraFd-EGFP |
| Tra3Fab | SP-8His-TraLC-linker36B-TraFd-linker20A-TraLC-linker36B-TraFd-linker20B-linker36B-TraFd-EGFP |
| TrascIgG60A | SP-8His-5L-TraLC-linker60-TraHC-EGFP |
| TrascIgG60B | SP-5L-TraLC-Iinker60-TraHC-EGFP-8His |

### Example 1: Plasmid construction

### 1. Gene synthesis

Specifically, TrascFab34, TrascFab60, TrascFab120, and TrascIgG60B were obtained by gene synthesis; TrascFab0, TrascFab6, TrascFab15, TrascFab25, TrascFab30, TrascFab180, TrascFab34A, TrascFab34B, TrascFab34C, TrascFab60A, TrascFab36A, TrascFab36B, TrascFab36B, and TrascFab82 were constructed from TrascFab60 by PCR using primers. The primer sequences are shown in Table 2 below.

| Table 2 | |
|---|---|
| Primer name | 5'-3' sequence |
| TrascFab0-F | TCAACAGAGGTGAATGTGAAGTTCAATTGGTTGAATCTGGTG |
| TrascFab0-R | ACATTCACCTCTGTTGAAAGACTTG |
| TrascFab6-F | TCTGGTGGTGGTTCTGGTGAAG |
| TrascFab6-R | ACCACCACCAGAACATTCACCTC |
| TrascFab82-F | CACTGGCTCTGGCGCT GGTTCTGAAGTTCAATTGGTTG |
| TrascFab82-R | AGCGCCAGAGCCAGTGCCAGCGCCGCCAGTAGAGCCAGCGCCGCCAGTAGAGCC AGCGCCGCCAGTAGAGCC AGCACCAGCGGTAGC |

### 2. Plasmid construction

Using TrascFab60 as a template, PCR was performed with the corresponding primers. The PCR product was digested with DpnI in an incubator at 37°C for at least 4 hours, followed by transformation (DH5α). Finally, single colonies were picked, and the plasmids were verified by sequencing. The plasmid structure is shown in FIG. 1.

### Example 2: Transformation of TrascFab60 plasmid

1 µL of the target plasmid was added to 20 µL of DH5α, placed on ice for 30 minutes, and heat-shocked at 42°C for 45 seconds. The mixture was placed on ice for 2 minutes, added with 500 µL of medium, and incubated at 37°C with shaking at 200 rpm for 1 hour. 100 µL of the culture was pipetted onto an LB plate (containing antibiotic) and incubated at 37°C in an inverted position for 12 to 16 hours. The plate was then removed and stored in a 4°C refrigerator.

### Example 3: Extraction of TrascFab60 plasmid (taking 10 mL of bacterial culture as an example)

(1) Collection of bacterial cells: The bacterial culture incubated for 12 to 16 hours was centrifuged at 4000 rpm for 10 minutes at 4°C. The supernatant was discarded, followed by brief centrifugation, and residual liquid on the surface of the pellet was removed using a pipette.
(2) 400 µL of P1 was added to the pellet, followed by vortexing for more than 2 minutes to completely disperse the bacterial pellet in P1.
(3) 500 µL of P2 was added to each sample, and the 2 mL EP tube was gently inverted 8 times.
(4) 700 µL of P3 was added to each sample, and the 2 mL EP tube was gently inverted 8 times.
(5) The mixture was centrifuged at 12,000 rpm for 15 minutes at 4°C to remove the resulting precipitate.
(6) The supernatant was taken and mixed with 100 µL of pre-shaken magnetic beads. The mixture was incubated at room temperature for 5 minutes, and the EP tube was inverted every 1 minute to ensure that the magnetic beads are fully mixed with the supernatant.
(7) The beads were captured on a magnetic rack and the supernatant was discarded (method: magnetic adsorption should last about 5 to 10 seconds; discard the supernatant once it appears slightly yellow and promptly remove the tube from the magnetic rack to prevent the beads from binding too tightly, which may hinder dispersion in the next step; since the plasmid conformation is easily disrupted by external forces, try to avoid vortexing during the washing process, as the quality of the plasmid conformation affects IVTT activity).
(8) The beads were washed with 1 mL of PR buffer. Since the beads were not tightly bound in the previous step, the beads were manually shaken for 1 minute. The supernatant was discarded using a magnetic rack as described above.
(9) The beads were washed with 1.2 mL of W buffer. Since the beads were not tightly bound in the previous step, the beads were manually shaken for 2 minutes. The supernatant was discarded using a magnetic rack as described above.
(10) Step 9 was repeated.
(11) The EP tube was placed on a magnetic rack, and the beads were dried with a hair dryer at minimum power for about 12 minutes. The completely dried beads appeared cracked, and no visible moisture was observed on the side of the EP tube where the beads adhered.
(12) The dried beads were gently scraped to the bottom of the EP tube using a pipette tip, followed by addition of 400 µL of elution buffer. The tube was vortexed for 15 seconds and gently shaken intermittently for up to 5 minutes to ensure complete elution. The supernatant was collected using a magnetic rack.
(13) The mixture was centrifuged at 12,000 rpm for 5 minutes at 4°C to remove residual beads.
(14) The supernatant was collected using a pipette, and centrifuged at 12,000 rpm for 10 minutes at 4°C to remove residual beads.
(15) The supernatant was collected, and the plasmid concentration was measured.
(16) 1% agarose gel electrophoresis was performed to observe the plasmid morphology, and the results are shown in FIG. 2.

### Example 4: Preparation of AMPI product of TrascFab60 (10 mL scale)

(1) 10 mL of AMPI buffer was prepared (the AMPI buffer used was the commercially available product AMPiX (10× DNA Amplifier) from Kangma-Healthcode (Shanghai) Biotech Co., Ltd., product model: PROTN_AMPiN10V03500).
**(2)** A certain amount of plasmid was added to achieve a final concentration of 4 ng/µL.
(3) 5 µL of AMPI enzyme was added.
(4) The mixture was mixed thoroughly and incubated at 30 rpm for 2 hours at 37°C. The resulting product mixture was then analyzed by 1% agarose gel electrophoresis. Using TrascFab60 as an example, the protein purification results are shown in FIG. 3.

### Example 5: Expression of TrascFab60 protein (250 mL scale)

8.3 mL of AMPI product was added to 250 mL of fast (the volume of AMPI product was 1/30 of that of fast). The mixture was incubated at 100 rpm in an incubator at 30 ± 2°C for 3 to 6 hours (fast is a commercially available *in vitro* cell-free synthesis system kit from Kangma-Healthcode (Shanghai) Biotech Co., Ltd., product model: profac_fast0510000, CoPure: FAST5 + His-Monster Beads + General Magnetic Rack). After the reaction was completed, 10 µL of the reaction mixture was added to a 384-well black plate, with three replicates per sample. The plate was immediately placed in a Tecan Infinite F200 microplate reader, with the excitation wavelength set to 488 nm, the emission wavelength set to 507 nm, and the manual gain set to 32. Readings were taken to measure expression activity, using the relative light unit (RLU) as the activity unit. The RFU of TrascFab60 was measured to be 1279.

### Example 6: Purification of TrascFab60 protein

(1) The volume of magnetic beads was determined to be 1% of the total volume of fast.
(2) The beads were washed three times with water before use, vortexed for 15 seconds each time, and collected using a magnetic rack. The volume of water used each time was 5 to 10 times the volume of the beads.
(3) Binding of target protein to beads: The washed beads were mixed with the IVTT product in a large beaker and incubated at 100 rpm for 1 hour at 4°C. The beads were then collected into a centrifuge tube.
(4) Washing of impurities: The protein-bound beads were washed three times with 10 mM imidazole at 180 rpm for 10 minutes each time. The volume of wash buffer used each time was 3 to 5 times the volume of the beads.
(5) Elution was performed with 250 mM imidazole elution buffer. The volume of the first elution was twice the volume of the beads, and the volume of the second elution was equal to the volume of the beads.
(6) Residual beads were removed by centrifugation. Specifically, the first centrifugation was performed at 12,000 rpm for 5 minutes, and the second centrifugation was performed at 12,000 rpm for 10 minutes. The purified protein was analyzed by SDS-PAGE, and the results are shown in FIG. 4.

### Example 7

The experimental methods described in Examples 1 to 6 were repeated to prepare the proteins TrascFab0, TrascFab6, TrascFab15, TrascFab25, TrascFab30, TrascFab180, TrascFab34A, TrascFab34B, TrascFab34C, TrascFab60A, TrascFab36A, TrascFab36B, TrascFab36B, TrascFab34, TrascFab82, TrascFab120, TrascFab180, TraFabHL, Tra2Fab, Tra3Fab, TrascIgG60A, and TrascIgG60A, respectively. FIG. 2 shows the electrophoretic analysis of the plasmids prepared in Examples 2 and 7 of the present disclosure, in which M represents the marker, and lanes 1 to 7 represent the plasmids TrascFab0, TrascFab6, TrascFab34, TrascFab60, TrascFab82, TrascFab120, and TrascIgG60, respectively. FIG. 3 shows the purification analysis of the AMPI products from Examples 2 and 7 of the present disclosure, in which M represents the marker, and lanes 1 to 7 represent the electrophoresis results of the AMPI products of TrascFab0, TrascFab6, TrascFab34, TrascFab60, TrascFab82, TrascFab120, and scIgG60, respectively.

After the reaction was completed, the expression activity was measured using the relative light unit (RLU) as the activity unit. The RFU results are shown in Table 3.

| Table 3 | | | |
|---|---|---|---|
| Protein name | RFU | Protein name | RFU |
| TrascFab0 | 1415 | TrascFab180 | 637 |
| TrascFab6 | 1373 | TrascFab34A | 1265 |
| TrascFab15 | 1260 | TrascFab34B | 1060 |
| TrascFab25 | 1126 | TrascFab34C | 995 |
| TrascFab30 | 1278 | TrascFab60A | 1102 |
| TrascFab34 | 984 | TrascFab36A | 1123 |
| TrascFab60 | 1279 | TrascFab36B | 1417 |
| TrascFab82 | 1330 | TrascFab36C | 1192 |
| TrascFab120 | 1190 | TrascFabHL | 1195 |
| Tra2Fab | 628 | Tra3Fab | 202 |
| TrascIgG60A | 330 | TrascIgG60B | 354 |

The results demonstrated that the single-chain antibodies of the present disclosure could be successfully expressed in an *in vitro* cell-free expression system.

### Example 8: ELISA detection of binding activity of TrascFab or scIgG to ErbB2 antigen

I. Establishment of ELISA detection method
1. The Trastuzumab-specific antigen was diluted to 0.25 µg/mL with coating buffer, added to an ELISA plate at 100 µL/well, and incubated overnight at 4°C.
2. The plate was washed three times with 300 µL of PBST per wash, followed by addition of 200 µL of blocking buffer, and incubated at 37°C for 1 hour.
3. The plate was washed three times with 300 µL of PBST per wash. The samples and commercially available monoclonal antibodies were diluted to 20 µg/mL with dilution buffer (PBST containing 1% BSA), followed by 3-fold serial dilution for a total of 11 dilutions. The diluted samples were added to the plate at 100 µL/well, with dilution buffer used as a blank control, and incubated at 37°C for 1 hour.
4. The plate was washed three times with 300 µL of PBST per wash, followed by addition of HRP-conjugated rabbit anti-human IgG (Fab specific) (1:5000 dilution) at 100 µL/well, and incubated at 37°C for 1 hour.
5. The plate was washed three times, followed by addition of TMB chromogenic substrate at 100 µL/well, and incubated at 37°C for 10 minutes. The reaction was terminated by addition of stop solution (2 M sulfuric acid) at 50 µL/well, and the absorbance was measured at 450 nm using a microplate reader.
6. Prism software was used to generate curves by selecting the 4-parameter logistic model, with sample concentration as the x-axis and A450 as the y-axis, and to calculate the EC50 values.

II. Detection results
1. According to the ELISA detection method established in this example, the purified proteins TrascFab0, TrascFab6, TrascFab15, TrascFab25, TrascFab30, TrascFab34, TrascFab60, TrascFab82, TrascFab120, and TrascFab180 were tested for their binding activity to ErbB2 antigen. Curves were plotted using the 4-parameter logistic model, and the results are shown in FIG. 5.
With sample concentration as the x-axis and A450 as the y-axis, the EC50 values were calculated as shown in Table 4.

| Table 4 | | |
|---|---|---|
| Protein name | EC50 (ng/mL) | EC50 (nM) |
| TrascFab0 | 309.9 | 3.98 |
| TrascFab6 | 139.2 | 1.78 |
| TrascFab15 | 83.19 | 1.05 |
| TrascFab25 | 52.52 | 0.66 |
| TrascFab30 | 43.31 | 0.54 |
| TrascFab34 | 42.83 | 0.53 |
| TrascFab60 | 40.22 | 0.49 |
| TrascFab82 | 37.08 | 0.44 |
| TrascFab120 | 40.07 | 0.46 |
| TrascFab180 | 40.35 | 0.44 |

The results demonstrated that the activity of the single-chain antibody of the present disclosure is closely related to the length of the linker. The activity of the single-chain antibody with a linker comprising less than 25 amino acid residues is significantly lower than that of the single-chain antibody with a linker comprising more than 25 amino acid residues. In particular, the activity of the single-chain antibody with a linker comprising 82 amino acid residues is more than 3 times and in some cases up to more than 9 times higher than that of TrascFab0 and TrascFab6.
2. Activity assay of purified TrascFab proteins with different linker lengths and compositions

According to the ELISA detection method established in this example, the purified proteins TrascFab34A, TrascFab34B, TrascFab34C, TrascFab60A, TrascFab36A, TrascFab36B, TrascFab36B, TrascFab36BHL, TrascIgG60A, and TrascIgG60B obtained in Example 7, as well as the commercially available monoclonal antibody Trastuzumab, were tested for their binding activity to ErbB2 antigen. Curves were plotted using the 4-parameter logistic model, with sample concentration as the x-axis and A450 as the y-axis, and the EC50 values were calculated as shown in Table 5.

| Table 5 | | |
|---|---|---|
| Protein name | EC50 (ng/mL) | EC50 (nM) |
| TrascFab34A | 125.1 | 1.53 |
| TrascFab34B | 74.99 | 0.90 |
| TrascFab34C | 559.6 | 6.70 |
| TrascFab60A | 296.1 | 3.48 |
| TrascFab36A | 92.45 | 1.14 |
| TrascFab36B | 59.84 | 0.74 |
| TrascFab36C | 78.59 | 0.98 |
| TrascFabHL | 75.72 | 0.94 |
| Tra3Fab | 72.04 | 0.40 |
| Tra2Fab | 83.33 | 0.64 |
| TrascIgG60A | 12.32 | 0.055 |
| TrascIgG60B | 12.82 | 0.06 |
| Trastuzumab | 9.505 | 0.065 |

The experimental results demonstrated that TrascFab34A, TrascFab34B, and TrascFab34C all exhibited significantly reduced activity compared to TrascFab34, indicating that the activity of the single-chain antibody is not only closely related to the length of the linker, but also closely related to the amino acid residue composition of the linker. The activity of the single-chain antibody with a linker primarily consisting of amino acid residues such as G, S, and E is significantly higher than that of the single-chain antibody with a linker primarily consisting of amino acid residues such as R, Y, E, I, and K. The finding was also confirmed by the binding affinity of TrascFab60A compared to TrascFab60.

Furthermore, the binding affinity of TrascFab36A, TrascFab36B, and TrascFab36C was also satisfactory, confirming that as long as the linker primarily comprises amino acid residues such as G, S, E, and A, together with a small number of other amino acid residues such as V, P, L, R, Y, and N, the binding affinity of the single-chain antibody will not be substantially affected.

The activity of TrascFabHL was essentially equivalent to that of TrascFab36C, confirming that the connection sequence of LC and Fd has little effect on the activity of the single-chain antibody. That is, different connection sequences of the light and heavy chains in the single-chain antibody of the present disclosure can achieve the purpose of the present disclosure.

The binding affinity of Tra3Fab and Tra2Fab was also satisfactory, indicating that tandem expression of Scfab can also achieve the purpose of the present disclosure and falls within the scope of protection of the present disclosure.

Furthermore, the single-chain antibodies TrascIgG60A and TrascIgG60B, which are similar to full-length antibodies, exhibited significantly enhanced activity, demonstrating that TrascIgG60A and TrascIgG60B obtained using the method of the present disclosure exhibit binding affinity essentially comparable to that of the commercially available monoclonal antibody Trastuzumab, which confirms that the method of the present disclosure is also applicable to single-chain antibodies prepared by linking full-length heavy and light chains.

The experimental results demonstrated that TrascIgG60A and TrascIgG60B exhibited high binding activity to ErbB2 antigen, which is comparable to that of the commercially available monoclonal antibody Trastuzumab. Compared to the commercially available monoclonal antibody Trastuzumab (natural double-chain structure), the single-chain antibody has unparalleled ease of expression. Therefore, while ensuring antibody activity, the single-chain antibody provided by the present disclosure addresses the drawbacks of low expression efficiency in traditional antibodies and limitations that hinder their commercial application. The single-chain antibody provided by the present disclosure possesses high expression efficiency, high affinity, and high stability, which can be produced on a large scale in combination with an *in vitro* cell-free protein synthesis system, thereby exhibiting good prospects for industrialization and commercialization.

It should be noted that the single-chain antibody of the Fab fragment did not achieve activity comparable to that of the commercially available monoclonal antibody Trastuzumab, which is reasonable since the antibody activity of the commercially available full-length antibody is inherently higher than that of the Fab fragment. For example, for the same linker60, the antibody activity of Fab (TrascFab60) was 0.49, while the activity of the full-length antibodies (TrascIgG60A and TrascIgG60B) was close to that of the commercially available monoclonal antibody Trastuzumab (0.055), demonstrating that the use of linker60 for connecting the light and heavy chains is reasonable, both for full-length antibodies and for Fab fragments.

Thus, under the premise that Fab fragment antibodies exhibit relatively similar binding activity, their full-length antibodies will also exhibit relatively similar binding activity. For example, based on the results in Table 4 where the activity is close to or less than that of TrascFab60 with linker60, it can be inferred that their full-length antibodies will also exhibit activity at least comparable to that of the commercially available full-length monoclonal antibody Trastuzumab.

### II. Experiments on single-chain antibodies constructed based on other types of antibodies

To verify the present disclosure, single-chain antibodies were synthesized using an *in vitro* synthesis system, including single-chain antibodies TisIgG60 and TisscFab60 designed based on Tislelizumab; single-chain antibodies DupIgG60 and DupscFab60 designed based on Dupilumab; single-chain antibodies AdaIgG60 and AdascFab60 designed based on Adalimumab; single-chain antibodies BevIgG60 and BevscFab60 designed based on Bevacizumab; single-chain antibodies DarIgG60 and DarscFab60 designed based on Daratumumab; single-chain antibodies OmaIgG60 and OmascFab60 designed based on Omalizumab; single-chain antibodies CetIgG60 and CetscFab60 designed based on Cetuximab; and single-chain antibody DenscFab36 designed based on Denosumab. The specific plasmid structures are shown in Table 6.

| Table 6 | |
|---|---|
| Protein name | Protein structure |
| TisIgG60 | SP-8His-5L-TisLC-linker60-TisHC-EGFP |
| TisscFab60 | SP-8His-5L-TisLC-linker60-TisFd-EGFP |
| DupIgG60 | SP-8His-5L-DupLC-linker60-DupHC-EGFP |
| DupscFab60 | SP-8His-5L-DupLC-linker60-DupFd-EGFP |
| AdaIgG60 | SP-8His-5L-AdaLC-linker60-AdaHC-EGFP |
| AdascFab60 | SP-8His-5L-AdaLC-linker60-AdaFd-EGFP |
| BevIgG60 | SP-8His-5L-BevLC-linker60-BevHC-EGFP |
| BevscFab60 | SP-8His-5L-BevLC-linker60-BevFd-EGFP |
| DarIgG60 | SP-8His-5L-DarLC-linker60-DarHC-EGFP |
| DarscFab60 | SP-8His-5L-DarLC-linker60-DarFd-EGFP |
| OmaIgG60 | SP-8His-5L-OmaLC-linker60-OmaHC-EGFP |
| OmascFab60 | SP-8His-5L-OmaLC-linker60-OmaFd-EGFP |
| CetIgG60 | SP-8His-5L-CetLC-linker60-CetHC-EGFP |
| CetscFab60 | SP-8His-5L-CetLC-linker60-CetFd-EGFP |
| DenscFab60 | SP-8His-5L-DenLC-linker60-DenFd-EGFP |

### Example 9

The preparation methods described in Examples 1 to 6 were repeated to prepare TisIgG60, TisscFab60, DupIgG60, DupscFab60, AdaIgG60, AdascFab60, BevIgG60, BevscFab60, DarIgG60, DarscFab60, OmaIgG60, OmascFab60, CetIgG60, and CetscFab60, respectively. After the reaction was completed, the reaction mixture was tested for expression activity and affinity.

The expression activity was measured using the relative light unit (RLU) as the activity unit. The RFU results are shown in Table 7.

| Table 7 | | | |
|---|---|---|---|
| Protein name | RFU | Protein name | RFU |
| TisIgG60 | 180 | DarIgG60 | 363 |
| TisscFab60 | 192.9 | DarscFab60 | 1184 |
| DupIgG60 | 323 | OmaIgG60 | 447 |
| DupscFab60 | 1299 | OmascFab60 | 1670 |
| AdaIgG60 | 214 | CetIgG60 | 227 |
| AdascFab60 | 374 | CetscFab60 | 967 |
| BevIgG60 | 192 | DenscFab60 | 1428 |
| BevscFab60 | 470 | | |

The results demonstrated that the preparation method for the single-chain antibody of the present disclosure is applicable to various types of antibodies, including IgG1, IgG2, and/or IgG4, all of which are suitable for the preparation method for the single-chain antibody of the present disclosure and exhibit favorable expression activity, and can be successfully expressed in an *in vitro* cell-free expression system.

### Example 10: ELISA detection of binding activity of TisIgG60 and TisscFab60 to recombinant human PD-1 protein

I. ELISA detection method
   1. The recombinant human PD-1 protein was diluted to 0.25 µg/mL with coating buffer, added to an ELISA plate at 100 µL/well, and incubated overnight at 4°C.
   2. The plate was washed three times with 300 µL of PBST per wash, followed by addition of 200 µL of blocking buffer, and blocked at 37°C for 1 hour.
   3. The plate was washed three times with 300 µL of PBST per wash. The samples and commercially available monoclonal antibodies were diluted to 20 µg/mL with dilution buffer (PBST containing 1% BSA), followed by 3-fold serial dilution for a total of 11 dilutions. The diluted samples were added to the plate at 100 µL/well, with dilution buffer used as a blank control, and incubated at 37°C for 1 hour.
   4. The plate was washed three times with 300 µL of PBST per wash, followed by addition of HRP-conjugated rabbit anti-human IgG (Fab specific) (1:5000 dilution) at 100 µL/well, and incubated at 37°C for 1 hour.
   5. The plate was washed three times, followed by addition of TMB chromogenic substrate at 100 µL/well, and incubated at 37°C for 10 minutes. The reaction was terminated by addition of stop solution (2 M sulfuric acid) at 50 µL/well, and the absorbance was measured at 450 nm using a microplate reader.
   6. Prism software was used to generate curves by selecting the 4-parameter logistic model, with sample concentration as the x-axis and A450 as the y-axis, and to calculate the EC50 values.
II. Detection results

According to the ELISA detection method established in this example, TisIgG60, TisscFab60, and Tislelizumab were tested for their binding activity to ErbB2 antigen. Curves were plotted using the 4-parameter logistic model, with sample concentration as the x-axis and A450 as the y-axis, and the EC50 values were calculated as shown in Table 8.

| Table 8 | | | |
|---|---|---|---|
| Protein name | TisIgG60 | TisscFab60 | Commercially available Tislelizumab |
| EC50 (ng/mL) | 16.18 | 192.9 | 14.26 |

The experimental results demonstrated that the preparation method for the single-chain antibody of the present disclosure is applicable to various types of antibodies, including IgG1, IgG2, and/or IgG4, all of which are suitable for the preparation method for the single-chain antibody of the present disclosure, exhibiting appropriate binding affinity and unparalleled ease of expression. Therefore, while ensuring antibody activity, the single-chain antibody provided by the present disclosure addresses the drawbacks of low expression efficiency in traditional antibodies and limitations that hinder their commercial application. The single-chain antibody provided by the present disclosure possesses high expression efficiency, high affinity, and high stability, which can be produced on a large scale in combination with an *in vitro* cell-free protein synthesis system, thereby exhibiting good prospects for industrialization and commercialization.

**The sequences mentioned above, along with their corresponding names and sequence numbers, are summarized in Table 9.**

| Table 9 | | |
|---|---|---|
| Name | Sequence No. | Amino acid sequence |
| SP | SEQ ID NO: 1 | MITETSSPFRSIFSHSGK |
| 8His | SEQ ID NO: 2 | HHHHHHHH |
| 5L | SEQ ID NO: 3 | GSGGS |
| TraLC | SEQ ID NO: 4 | DIQMTQSPSSLSASVGDRVTITCRASQDVNTAVAWYQQKPGKAPKL LIYSASFLYSGVPSRFSGSRSGTDFTLTISSLQPEDFATYYCQQHYTT PPTFGQGTKVEIKRTVAAPSVFIFPPSDEQLKSGTASVVCLLNNFYPR EAKVQWKVDNALQSGNSQESVTEQDSKDSTYSLSSTLTLSKADYEK HKVYACEVTHQGLSSPVTKSFNRGEC |
| TraFd | SEQ ID NO: 5 | EVQLVESGGGLVQPGGSLRLSCAASGFNIKDTYIHWVRQAPGKGLE WVARIYPTNGYTRYADSVKGRFTISADTSKNTAYLQMNSLRAEDTA VYYCSRWGGDGFYAMDYWGQGTLVTVSSASTKGPSVFPLAPSSKS TSGGTAALGCLVKDYFPEPVTVSWNSGALTSGVHTFPAVLQSSGLYS LSSVVTVPSSSLGTQTYICNVNHKPSNTKVDKKVEPKSCDK |
| TraHC | SEQ ID NO: 6 | EVQLVESGGGLVQPGGSLRLSCAASGFNIKDTYIHWVRQAPGKGLE WVARIYPTNGYTRYADSVKGRFTISADTSKNTAYLQMNSLRAEDTA VYYCSRWGGDGFYAMDYWGQGTLVTVSSASTKGPSVFPLAPSSKS TSGGTAALGCLVKDYFPEPVTVSWNSGALTSGVHTFPAVLQSSGLYS LSSVVTVPSSSLGTQTYICNVNHKPSNTKVDKKVEPKSCDKTHTCPP CPAPELLGGPSVFLFPPKPKDTLMISRTPEVTCVVVDVSHEDPEVKF NWYVDGVEVHNAKTKPREEQYNSTYRVVSVLTVLHQDWLNGKEYK CKVSNKALPAPIEKTISKAKGQPREPQVYTLPPSREEMTKNQVSLTC LVKGFYPSDIAVEWESNGQPENNYKTTPPVLDSDGSFFLYSKLTVDK SRWQQGNVFSCSVMHEALHNHYTQKSLSLSPGK |
| 10L | SEQ ID NO: 7 | TGGAGTGSGA |
| EGFP | SEQ ID NO: 8 | VSKGEELFTGVVPILVELDGDVNGHKFSVRGEGEGDATNGKLTLKFI CTTGKLPVPWPTLVTTLTYGVQCFSRYPDHMKQHDFFKSAMPEGYV QERTISFKDDGTYKTRAEVKFEGDTLVNRIELKGIDFKEDGNILGHK LEYNFNSHNVYITADKQKNGIKANFKIRHNVEDGSVQLADHYQQNT PIGDGPVLLPDNHYLSTQSKLSKDPNEKRDHMVLLEFVTAAGITLGM DELYK |
| Linker6 | SEQ ID NO: 9 | SGGGSG |
| Linker15 | SEQ ID NO: 10 | GGGGSGGGGSGGGGS |
| Linker25 | SEQ ID NO: 11 | GGGGSGGGGSGGGGSGGGGSGGGGS |
| Linker20A | SEQ ID NO: 12 | GGGGSGGGGSGGGGSGGGGS |
| Linker20B | SEQ ID NO: 13 | GGSGGSGGSGGSGGSGGSGG |
| Linker30 | SEQ ID NO: 14 | SGGGSGGGSEGGGSEGGGSEGGGSEGGGSG |
| Linker34 | SEQ ID NO: 15 | SGGGSGGGSEGGGSEGGGSEGGGSEGGGSGGGSG |
| Linker34A | SEQ ID NO: 16 | RRYEERRYEERRYEERRYEERRYEERRYEERRYE |
| Linker34B | SEQ ID NO: 17 | YYEYYYYEYYYYEYYYYEYYYYEYYYYEYYEYYE |
| Linker34C | SEQ ID NO: 18 | IKYLEFISEAIIHVLHSRHPGDFGADAQGAMNKA |
| Linker60 | SEQ ID NO: 19 | GGSSGSGSGSTGTSSSGTGTSAGTTGTSASTSGSGSGGGGGSGGG GSAGGTATAGASSGS |
| Linker60A | SEQ ID NO: 20 | LAQSHATKHKIPIKYLEFISEAIIHVLHSRHPGDFGADAQGAMNKAL ELFRKDIAAKYKE |
| Linker82 | SEQ ID NO: 21 | GGSSGSGSGSTGTSSSGTGTSAGTTGTSASTSGSGSGGGGGSGGG GSAGGTATAGAGSTGGAGSTGGAGSTGGAGTGSGAGS |
| Linker120 | SEQ ID NO: 22 | GGSSGSGSGSTGTSSSGTGTSAGTTGTSASTSGSGSGGGGGSGGG GSAGGTATGGSSGGSGGSSGSGSGSTGTSSSGTGTSAGTTGTSAST SGSGSGGGGGSGGGGSAGGTATAGASSGS |
| Linker180 | SEQ ID NO: 23 | GGSSGSGSGSTGTSSSGTGTSAGTTGTSASTSGSGSGGGGGSGGG GSAGGTATAGASSGSGGSSGSGSGSTGTSSSGTGTSAGTTGTSAST SGSGSGGGGGSGGGGSAGGTATAGASSGSGGSSGSGSGSTGTSSS GTGTSAGTTGTSASTSGSGSGGGGGSGGGGSAGGTATAGASSGS |
| Linker36A | SEQ ID NO: 24 | GSGVPSRFSGSGSGTDFTLTISSLQPEDFATYYSGS |
| Linker36B | SEQ ID NO: 25 | GSGEVQLVESGGGLVQPGGSLRLSSAASGFNIKGSG |
| Linker36C | SEQ ID NO: 26 | GAGAGSGAGAGSGAGAGSGAGAGSGAGAGSGAGAGS |
| TisLC | SEQ ID NO: 27 | DIVMTQSPDSLAVSLGERATINCKSSESVSNDVAWYQQKPGQPPKL LINYAFHRFTGVPDRFSGSGYGTDFTLTISSLQAEDVAVYYCHQAYS SPYTFGQGTKLEIKRTVAAPSVFIFPPSDEQLKSGTASVVCLLNNFYP REAKVQWKVDNALQSGNSQESVTEQDSKDSTYSLSSTLTLSKADYE KHKVYACEVTHQGLSSPVTKSFNRGEC |
| TisHC | SEQ ID NO: 28 | QVQLQESGPGLVKPSETLSLTCTVSGFSLTSYGVHWIRQPPGKGLE WIGVIYADGSTNYNPSLKSRVTISKDTSKNQVSLKLSSVTAADTAVY YCARAYGNYWYIDVWGQGTTVTVSSASTKGPSVFPLAPCSRSTSES TAALGCLVKDYFPEPVTVSWNSGALTSGVHTFPAVLQSSGLYSLSSV VTVPSSSLGTKTYTCNVDHKPSNTKVDKRVESKYGPPCPPCPAPPVA GGPSVFLFPPKPKDTLMISRTPEVTCVVVAVSQEDPEVQFNWYVDG VEVHNAKTKPREEQFNSTYRVVSVLTVVHQDWLNGKEYKCKVSNK GLPSSIEKTISKAKGQPREPQVYTLPPSQEEMTKNQVSLTCLVKGFY PSDIAVEWESNGQPENNYKTTPPVLDSDGSFFLYSKLTVDKSRWQE GNVFSCSVMHEALHNHYTQKSLSLSLGKTGGAGTGSGA |
| TisFd | SEQ ID NO: 29 | QVQLQESGPGLVKPSETLSLTCTVSGFSLTSYGVHWIRQPPGKGLE WIGVIYADGSTNYNPSLKSRVTISKDTSKNQVSLKLSSVTAADTAVY YCARAYGNYWYIDVWGQGTTVTVSSASTKGPSVFPLAPCSRSTSES TAALGCLVKDYFPEPVTVSWNSGALTSGVHTFPAVLQSSGLYSLSSV VTVPSSSLGTKTYTCNVDHKPSNTKVDKRVESK |
| DupLC | SEQ ID NO: 30 | DIVMTQSPLSLPVTPGEPASISCRSSQSLLYSIGYNYLDWYLQKSGQ SPQLLIYLGSNRASGVPDRFSGSGSGTDFTLKISRVEAEDVGFYYCM QALQTPYTFGQGTKLEIKRTVAAPSVFIFPPSDEQLKSGTASVVCLLN NFYPREAKVQWKVDNALQSGNSQESVTEQDSKDSTYSLSSTLTLSK ADYEKHKVYACEVTHQGLSSPVTKSFNRGEC |
| DupHC | SEQ ID NO: 31 | EVQLVESGGGLEQPGGSLRLSCAGSGFTFRDYAMTWVRQAPGKGL EWVSSISGSGGNTYYADSVKGRFTISRDNSKNTLYLQMNSLRAEDT AVYYCAKDRLSITIRPRYYGLDVWGQGTTVTVSSASTKGPSVFPLAP CSRSTSESTAALGCLVKDYFPEPVTVSWNSGALTSGVHTFPAVLQSS GLYSLSSVVTVPSSSLGTKTYTCNVDHKPSNTKVDKRVESKYGPPCP PCPAPEFLGGPSVFLFPPKPKDTLMISRTPEVTCVVVDVSQEDPEVQ FNWYVDGVEVHNAKTKPREEQFNSTYRVVSVLTVLHQDWLNGKEY KCKVSNKGLPSSIEKTISKAKGQPREPQVYTLPPSQEEMTKNQVSLT CLVKGFYPSDIAVEWESNGQPENNYKTTPPVLDSDGSFFLYSRLTVD KSRWQEGNVFSCSVMHEALHNHYTQKSLSLSLG |
| DupFd | SEQ ID NO: 32 | EVQLVESGGGLEQPGGSLRLSCAGSGFTFRDYAMTWVRQAPGKGL EWVSSISGSGGNTYYADSVKGRFTISRDNSKNTLYLQMNSLRAEDT AVYYCAKDRLSITIRPRYYGLDVWGQGTTVTVSSASTKGPSVFPLAP CSRSTSESTAALGCLVKDYFPEPVTVSWNSGALTSGVHTFPAVLQSS GLYSLSSVVTVPSSSLGTKTYTCNVDHKPSNTKVDKRVESKYG |
| AdaLC | SEQ ID NO: 33 | DIQMTQSPSSLSASVGDRVTITCRASQGIRNYLAWYQQKPGKAPKL LIYAASTLQSGVPSRFSGSGSGTDFTLTISSLQPEDVATYYCQRYNR APYTFGQGTKVEIKRTVAAPSVFIFPPSDEQLKSGTASVVCLLNNFYP REAKVQWKVDNALQSGNSQESVTEQDSKDSTYSLSSTLTLSKADYE KHKVYACEVTHQGLSSPVTKSFNRGEC |
| AdaHC | SEQ ID NO: 34 | EVQLVESGGGLVQPGRSLRLSCAASGFTFDDYAMHWVRQAPGKGL EWVSAITWNSGHIDYADSVEGRFTISRDNAKNSLYLQMNSLRAEDT AVYYCAKVSYLSTASSLDYWGQGTLVTVSSASTKGPSVFPLAPSSKS TSGGTAALGCLVKDYFPEPVTVSWNSGALTSGVHTFPAVLQSSGLYS LSSVVTVPSSSLGTQTYICNVNHKPSNTKVDKKVEPKSCDKTHTCPP CPAPELLGGPSVFLFPPKPKDTLMISRTPEVTCVVVDVSHEDPEVKF NWYVDGVEVHNAKTKPREEQYNSTYRVVSVLTVLHQDWLNGKEYK CKVSNKALPAPIEKTISKAKGQPREPQVYTLPPSRDELTKNQVSLTCL VKGFYPSDIAVEWESNGQPENNYKTTPPVLDSDGSFFLYSKLTVDKS RWQQGNVFSCSVMHEALHNHYTQKSLSLSPGKTGGAGTGSGA |
| AdaFd | SEQ ID NO: 35 | EVQLVESGGGLVQPGRSLRLSCAASGFTFDDYAMHWVRQAPGKGL EWVSAITWNSGHIDYADSVEGRFTISRDNAKNSLYLQMNSLRAEDT AVYYCAKVSYLSTASSLDYWGQGTLVTVSSASTKGPSVFPLAPSSKS |
| | | TSGGTAALGCLVKDYFPEPVTVSWNSGALTSGVHTFPAVLQSSGLYS LSSVVTVPSSSLGTQTYICNVNHKPSNTKVDKKVEPKSCDK |
| BevLC | SEQ ID NO: 36 | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADYE KHKVYACEVTHQGLSSPVTKSFNRGEC |
| BevHC | SEQ ID NO: 37 | EVQLVESGGGLVQPGGSLRLSCAASGYTFTNYGMNWVRQAPGKGL EWVGWINTYTGEPTYAADFKRRFTFSLDTSKSTAYLQMNSLRAEDT AVYYCAKYPHYYGSSHWYFDVWGQGTLVTVSSASTKGPSVFPLAPS SKSTSGGTAALGCLVKDYFPEPVTVSWNSGALTSGVHTFPAVLQSSG LYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEV KFNWYVDGVEVHNAKTKPREEQYNSTYRVVSVLTVLHQDWLNGKE YKCKVSNKALPAPIEKTISKAKGQPREPQVYTLPPSREEMTKNQVSL TCLVKGFYPSDIAVEWESNGQPENNYKTTPPVLDSDGSFFLYSKLTV DKSRWQQGNVFSCSVMHEALHNHYTQKSLSLSPGKTGGAGTGSG A |
| BevFd | SEQ ID NO: 38 | EVQLVESGGGLVQPGGSLRLSCAASGYTFTNYGMNWVRQAPGKGL EWVGWINTYTGEPTYAADFKRRFTFSLDTSKSTAYLQMNSLRAEDT AVYYCAKYPHYYGSSHWYFDVWGQGTLVTVSSASTKGPSVFPLAPS SKSTSGGTAALGCLVKDYFPEPVTVSWNSGALTSGVHTFPAVLQSSG LYSLSSVVTVPSSSLGTQTYICNVNHKPSNTKVDKKVEPKSCDK |
| DarLC | SEQ ID NO: 39 | EIVLTQSPATLSLSPGERATLSCRASQSVSSYLAWYQQKPGQAPRLLI YDASNRATGIPARFSGSGSGTDFTLTISSLEPEDFAVYYCQQRSNWP PTFGQGTKVEIKRTVAAPSVFIFPPSDEQLKSGTASVVCLLNNFYPRE AKVQWKVDNALQSGNSQESVTEQDSKDSTYSLSSTLTLSKADYEKH KVYACEVTHQGLSSPVTKSFNRGEC |
| DarHC | SEQ ID NO: 40 | EVQLLESGGGLVQPGGSLRLSCAVSGFTFNSFAMSWVRQAPGKGLE WVSAISGSGGGTYYADSVKGRFTISRDNSKNTLYLQMNSLRAEDTA VYFCAKDKILWFGEPVFDYWGQGTLVTVSSASTKGPSVFPLAPSSKS TSGGTAALGCLVKDYFPEPVTVSWNSGALTSGVHTFPAVLQSSGLYS LSSVVTVPSSSLGTQTYICNVNHKPSNTKVDKRVEPKSCDKTHTCPP CPAPELLGGPSVFLFPPKPKDTLMISRTPEVTCVVVDVSHEDPEVKF NWYVDGVEVHNAKTKPREEQYNSTYRVVSVLTVLHQDWLNGKEYK CKVSNKALPAPIEKTISKAKGQPREPQVYTLPPSREEMTKNQVSLTC LVKGFYPSDIAVEWESNGQPENNYKTTPPVLDSDGSFFLYSKLTVDK SRWQQGNVFSCSVMHEALHNHYTQKSLSLSPGK |
| DarFd | SEQ ID NO: 41 | EVQLLESGGGLVQPGGSLRLSCAVSGFTFNSFAMSWVRQAPGKGLE WVSAISGSGGGTYYADSVKGRFTISRDNSKNTLYLQMNSLRAEDTA VYFCAKDKILWFGEPVFDYWGQGTLVTVSSASTKGPSVFPLAPSSKS TSGGTAALGCLVKDYFPEPVTVSWNSGALTSGVHTFPAVLQSSGLYS LSSVVTVPSSSLGTQTYICNVNHKPSNTKVDKRVEPKSCDK |
| OmaLC | SEQ ID NO: 42 | DIQLTQSPSSLSASVGDRVTITCRASQSVDYDGDSYMNWYQQKPG KAPKLLIYAASYLESGVPSRFSGSGSGTDFTLTISSLQPEDFATYYCQ QSHEDPYTFGQGTKVEIKRTVAAPSVFIFPPSDEQLKSGTASVVCLL NNFYPREAKVQWKVDNALQSGNSQESVTEQDSKDSTYSLSSTLTLS KADYEKHKVYACEVTHQGLSSPVTKSFNR |
| OmaHC | SEQ ID NO: 43 | EVQLVESGGGLVQPGGSLRLSCAVSGYSITSGYSWNWIRQAPGKGL EWVASITYDGSTNYADSVKGRFTISRDDSKNTFYLQMNSLRAEDTA VYYCARGSHYFGHWHFAVWGQGTLVTVSSGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKAEPKSCDKTHTCPPCPA PELLGGPSVFLFPPKPKDTLMISRTPEVTCVVVDVSHEDPEVKFNWY VDGVEVHNAKTKPREEQYNSTYRVVSVLTVLHQDWLNGKEYKCKV SNKALPAPIEKTISKAKGQPREPQVYTLPPSRDELTKNQVSLTCLVKG FYPSDIAVEWESNGQPENNYKTTPPVLDSDGSFFLYSKLTVDKSRW QQGNVFSCSVMHEALHNHYTQKSLSLSPGK |
| OmaFd | SEQ ID NO: 44 | EVQLVESGGGLVQPGGSLRLSCAVSGYSITSGYSWNWIRQAPGKGL EWVASITYDGSTNYADSVKGRFTISRDDSKNTFYLQMNSLRAEDTA VYYCARGSHYFGHWHFAVWGQGTLVTVSSGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKAEPKSCDK |
| CetLC | SEQ ID NO: 45 | DVQVTQSPSSLSASVGDRVTITCRSSQSLANSYGNTFLSWYLHKPG KAPQLLIYGISNRFSGVPDRFSGSGSGTDFTLTISSLQPEDFATYYCL QGTHQPYTFGQGTKVEIKRTVAAPSVFIFPPSDEQLKSGTASVVCLL NNFYPREAKVQWKVDNALQSGNSQESVTEQDSKDSTYSLSSTLTLS KADYEKHKVYACEVTHQGLSSPVTKSFNRGEC |
| CetHC | SEQ ID NO: 46 | EVQLVQSGAEVKKPGASVKVSCKASGYRFTNYWIHWVRQAPGQGL EWIGGINPGNNYATYRRKFQGRVTMTADTSTSTVYMELSSLRSEDT AVYYCTREGYGNYGAWFAYWGQGTLVTVSSASTKGPSVFPLAPCSR STSESTAALGCLVKDYFPEPVTVSWNSGALTSGVHTFPAVLQSSGLY SLSSVVTVPSSSLGTKTYTCNVDHKPSNTKVDKRVESKYGPPCPPCP APEFLGGPSVFLFPPKPKDTLMISRTPEVTCVVVDVSQEDPEVQFNW YVDGVEVHNAKTKPREEQFNSTYRVVSVLTVLHQDWLNGKEYKCK VSNKGLPSSIEKTISKAKGQPREPQVYTLPPSQEEMTKNQVSLTCLV KGFYPSDIAVEWESNGQPENNYKTTPPVLDSDGSFFLYSRLTVDKSR WQEGNVFSCSVMHEALHNHYTQKSLSLSLGK |
| CetFd | SEQ ID NO: 47 | EVQLVQSGAEVKKPGASVKVSCKASGYRFTNYWIHWVRQAPGQGL EWIGGINPGNNYATYRRKFQGRVTMTADTSTSTVYMELSSLRSEDT AVYYCTREGYGNYGAWFAYWGQGTLVTVSSASTKGPSVFPLAPCSR STSESTAALGCLVKDYFPEPVTVSWNSGALTSGVHTFPAVLQSSGLY SLSSVVTVPSSSLGTKTYTCNVDHKPSNTKVDKRVES |
| DenLC | SEQ ID NO: 48 | EIVLTQSPGTLSLSPGERATLSCRASQSVRGRYLAWYQQKPGQAPR LLIYGASSRATGIPDRFSGSGSGTDFTLTISRLEPEDFAVFYCQQYGS SPRTFGQGTKVEIKRTVAAPSVFIFPPSDEQLKSGTASVVCLLNNFYP |
| | | REAKVQWKVDNALQSGNSQESVTEQDSKDSTYSLSSTLTLSKADYE KHKVYACEVTHQGLSSPVTKSFNRGEC |
| DenHC | SEQ ID NO: 49 | EVQLLESGGGLVQPGGSLRLSCAASGFTFSSYAMSWVRQAPGKGLE WVSGITGSGGSTYYADSVKGRFTISRDNSKNTLYLQMNSLRAEDTA VYYCAKDPGTTVIMSWFDPWGQGTLVTVSSASTKGPSVFPLAPSSK STSGGTAALGCLVKDYFPEPVTVSWNSGALTSGVHTFPAVLQSSGLY SLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKF NWYVDGVEVHNAKTKPREEQYNSTYRVVSVLTVLHQDWLNGKEYK CKVSNKALPAPIEKTISKAKGQPREPQVYTLPPSRDELTKNQVSLTCL VKGFYPSDIAVEWESNGQPENNYKTTPPVLDSDGSFFLYSKLTVDKS RWQQGNVFSCSVMHEALHNHYTQKSLSLSPGK |
| DenFd | SEQ ID NO: 50 | EVQLLESGGGLVQPGGSLRLSCAASGFTFSSYAMSWVRQAPGKGLE WVSGITGSGGSTYYADSVKGRFTISRDNSKNTLYLQMNSLRAEDTA VYYCAKDPGTTVIMSWFDPWGQGTLVTVSSASTKGPSVFPLAPSSK STSGGTAALGCLVKDYFPEPVTVSWNSGALTSGVHTFPAVLQSSGLY SLSSVVTVPSSSLGTQTYICNVNHKPSNTKVDKKVEPKSCDK |

The preferred embodiments of the present disclosure have been described in detail above. It should be understood that those of ordinary skill in the art can make various modifications and variations to the present disclosure based on the concept of the present disclosure without creative effort. Therefore, any technical solutions that can be obtained by those skilled in the art through logical analysis, reasoning, or limited experimentation, on the basis of the prior art and in line with the concept of the present disclosure, shall fall within the scope of protection defined by the claims.

## Claims

1. A single-chain antibody, wherein the single-chain antibody is formed by connecting a light chain or light chain fragment of an antibody to a heavy chain or heavy chain fragment of an antibody via a linker; the linker comprises at least 20 amino acid residues, preferably 25 to 180 amino acid residues, and more preferably 34 to 120 amino acid residues;
preferably, the antibody is selected from IgM, IgG, IgA, IgD, or IgE.

2. The single-chain antibody according to claim 1, wherein the amino acid comprised in the linker is selected from serine (S), glycine (G), glutamic acid (E), threonine (T), alanine (A), arginine (R), tyrosine (Y), isoleucine (I), lysine (K), leucine (L), glutamine (Q), histidine (H), phenylalanine (F), valine (V), proline (P), aspartic acid (D), methionine (M), and asparagine (N); preferably, the amino acids comprised in the linker are selected from serine (S), glycine (G), glutamic acid (E), threonine (T), and alanine (A);
further preferably, the total number of serine (S), glycine (G), glutamic acid (E), threonine (T), and alanine (A) comprised in the linker accounts for 100%, at least 99%, at least 98%, at least 97%, at least 96%, at least 95%, at least 90%, at least 85%, at least 80%, at least 75%, at least 70%, at least 65%, at least 60%, at least 55%, and at least 50% of the total number of amino acids in the linker; further preferably, the total number of serine (S), glycine (G), and glutamic acid (E) comprised in the linker accounts for 100%, at least 99%, at least 98%, at least 97%, at least 96%, at least 95%, at least 90%, at least 85%, at least 80%, at least 75%, at least 70%, at least 65%, at least 60%, at least 55%, and at least 50% of the total number of amino acids in the linker; more preferably, the total number of serine (S), glycine (G), and glutamic acid (E) comprised in the linker accounts for 100%, at least 99%, at least 98%, at least 97%, at least 96%, at least 95%, at least 90%, at least 85%, at least 80%, at least 75%, at least 70%, at least 65%, at least 60%, at least 55%, and at least 50% of the total number of amino acids in the linker;
more preferably, the linker is one or more selected from the following: linker25 (SEQ ID NO: 11), linker30 (SEQ ID NO: 14), linker34 (SEQ ID NO: 15), linker34A (SEQ ID NO: 16), linker34B (SEQ ID NO: 17), linker34C (SEQ ID NO: 18), linker60 (SEQ ID NO: 19), linker60A (SEQ ID NO: 20), linker82 (SEQ ID NO: 21), linker120 (SEQ ID NO: 22), linker180 (SEQ ID NO: 23), linker36A (SEQ ID NO: 24), linker36B (SEQ ID NO: 25), linker36C (SEQ ID NO: 26), and/or a sequence having one or several amino acid substitutions, deletions, or additions relative to any one of the amino acid sequences described above, wherein the number of the amino acid substitutions, deletions, or additions may be 1 to 10, preferably 1 to 5.

3. The single-chain antibody according to any one of claims 1 to 2, wherein the IgG antibody is any one or more selected from IgG1, IgG2, IgG3, and IgG4;
the antibody is selected from an IgG1 antibody; preferably, the IgG1 antibody is any one or more of Trastuzumab, Bevacizumab, Daratumumab, Omalizumab, Cetuximab, Adalimumab, Ustekinumab, Ocrelizumab, Infliximab, Envafolimab, Atlizumab, and Atezolizumab;
the IgG2 antibody is any one or more of Denosumab, Erenumab, Tremelimumab, and Evocumab;
the IgG4 antibody is any one or more of Tislelizumab, Dupilumab, Rituximab, Pembrolizumab, Nivolumab, and Palivizumab.

4. The single-chain antibody according to any one of claims 1 to 3, wherein the antibody light chain is selected from a κ chain and/or a λ chain; the antibody light chain fragment is selected from a κ chain fragment and/or a λ chain fragment; the heavy chain is any one or more selected from a µ chain, a γ chain, an a chain, a δ chain, and an ε chain; the heavy chain fragment is any one or more selected from a µ chain fragment, a γ chain fragment, an a chain fragment, a δ chain fragment, and an ε chain fragment;
preferably, the antibody light chain is derived from Tislelizumab light chain TisLC or a fragment thereof, and the antibody heavy chain is derived from Tislelizumab heavy chain TisHC or a fragment TisFd thereof; or
the antibody light chain is derived from Dupilumab light chain DupLC or a fragment thereof, and the antibody heavy chain is derived from Dupilumab heavy chain DupHC or a fragment DupFd thereof; or
the antibody light chain is derived from Adalimumab light chain AdaLC or a fragment thereof, and the antibody heavy chain is derived from Adalimumab heavy chain AdaHC or a fragment AdaFd thereof; or
the antibody light chain is derived from Bevacizumab light chain BevLC or a fragment thereof, and the antibody heavy chain is derived from Bevacizumab heavy chain BevHC or a fragment BevFd thereof; or
the antibody light chain is derived from Daratumumab light chain DarLC or a fragment thereof, and the antibody heavy chain is derived from Daratumumab heavy chain DarHC or a fragment DarFd thereof; or
the antibody light chain is derived from Omalizumab light chain OmaLC or a fragment thereof, and the antibody heavy chain is derived from Omalizumab heavy chain OmaHC or a fragment OmaFd thereof; or
the antibody light chain is derived from Cetuximab light chain CetLC or a fragment thereof, and the antibody heavy chain is derived from Cetuximab heavy chain CetHC or a fragment CetFd thereof; or
the antibody light chain is derived from Denosumab light chain DenLC or a fragment thereof, and the antibody heavy chain is derived from Denosumab heavy chain DenHC or a fragment DenFd thereof;
the antibody light chain is derived from Trastuzumab light chain TraLC or a fragment thereof, and the antibody heavy chain is derived from Trastuzumab heavy chain TraHC or a fragment thereof; or
the antibody light chain is Trastuzumab light chain TraLC, and the antibody heavy chain fragment is a Trastuzumab heavy chain fragment TraFd.

5. The single-chain antibody according to any one of claims 1 to 4, wherein the single-chain antibody further comprises a signal peptide SP; preferably, the single-chain antibody is connected at the N-terminus of the light chain or light chain fragment with the signal peptide SP to improve the expression efficiency and expression level of the single-chain antibody; preferably, the SP comprises the amino acid sequence shown in SEQ ID NO: 1 or a sequence having one or more amino acid substitutions, deletions, or additions relative to the sequence shown in SEQ ID NO: 1; more preferably, the number of the amino acid substitutions, deletions, or additions is 1 to 10.

6. The single-chain antibody according to any one of claims 1 to 5, wherein the single-chain antibody further comprises a fluorescent protein tag; preferably, the single-chain antibody is connected at the C-terminus of the heavy chain or heavy chain fragment with the fluorescent protein tag; more preferably, the fluorescent protein tag comprises eGFP, or a protein tag improved based on eGFP, such as mTagBFP2, moxCerulean3, AmCyanl, MiCy, ZsGreen, Clover, mVenus, ZsYellow1, mKO2, TurboRFP, tdTomato, eqFP611, mKate1.3, mNeptune2, miRFP670, mAmetrine, PAmCherry2, and mEos3.2; more preferably, the eGFP comprises the amino acid sequence shown in SEQ ID NO: 8, or a sequence having one or more amino acid substitutions, deletions, or additions relative to the amino acid sequence shown in SEQ ID NO: 8; preferably, the number of the amino acid substitutions, deletions, or additions is 1 to 20.

7. The single-chain antibody according to any one of claims 1 to 6, wherein the single-chain antibody further comprises a tag label; preferably, the single-chain antibody is connected at the N-terminus of the light chain or light chain fragment with the tag label; more preferably, the tag label is a His tag; preferably, the number of histidine residues in the His tag is 3 to 15.

8. The single-chain antibody according to any one of claims 1 to 7, wherein the fluorescent protein tag, the tag label, or the signal peptide is connected to the light chain, light chain fragment, heavy chain, or heavy chain fragment of the antibody via a single bond or a flexible linker; preferably, the flexible linker comprises 1 to 20 amino acid residues; preferably, the flexible linker is 5L (SEQ ID NO: 3: GSGGS) and/or 10L (SEQ ID NO: 7: TGGAGTGSGA).

9. A one or more isolated nucleic acids, wherein the nucleic acid encodes the single-chain antibody according to any one of claims 1 to 12; preferably, the nucleic acid is a DNA sequence and/or an RNA sequence.

10. A one or more expression vectors, wherein the vector comprises a nucleic acid encoding the single-chain antibody according to any one of claims 1 to 12; preferably, the expression vector comprises, but is not limited to, a eukaryotic plasmid vector, a eukaryotic viral vector, a prokaryotic plasmid, a specific vector, a shuttle vector, a minichromosome, and various vectors; preferably, the expression vector is a eukaryotic plasmid expression vector and a prokaryotic plasmid expression vector.

11. A host cell, wherein the host cell contains the vector according to claim 10, or has the nucleic acid according to claim 9 integrated into its genome; the host cell is selected from a prokaryotic host cell and/or a eukaryotic host cell; preferably, the host cell is a eukaryotic cell, such as yeast, a progenitor cell, a Chinese hamster ovary cell, an insect cell, a wheat germ cell, and a rabbit reticulocyte; more preferably, the yeast is one or more selected from the group consisting of *Saccharomyces cerevisiae* and one or more yeasts of the *Kluyveromyces;* in another preferred embodiment, the *Kluyveromyces* is one or more selected from the group consisting of *Kluyveromyces lactis, Kluyveromyces marxianus,* and *Kluyveromyces dobzhanskii.*

12. An *in vitro* cell-free protein synthesis system, wherein the *in vitro* cell-free protein synthesis system comprises the nucleic acid according to claim 9 or the expression vector according to claim 10, or a lysate or extract of the host cell according to claim 17;
the *in vitro* cell-free protein synthesis system is capable of expressing the single-chain antibody according to any one of claims 1 to 8 under conditions suitable for expression;
preferably, the *in vitro* cell-free protein synthesis system comprises, but is not limited to, an *E. coli in vitro* protein synthesis system, a bacterial *in vitro* protein synthesis system, a mammalian cell *in vitro* protein synthesis system, a plant cell *in vitro* protein synthesis system, a yeast cell *in vitro* protein synthesis system, and an insect cell *in vitro* protein synthesis system; preferably a yeast *in vitro* protein synthesis system, more preferably a *Kluyveromyces in vitro* protein synthesis system, and most preferably a *Kluyveromyces lactis* or *Kluyveromyces marxianus in vitro* protein synthesis system.

13. The *in vitro* cell-free protein synthesis system according to claim 12, wherein the *in vitro* protein synthesis system comprises a cell extract, tris(hydroxymethyl)aminomethane, potassium acetate, magnesium acetate, a mixture of nucleoside triphosphates (NTPs), and a mixture of amino acids.

14. Use of the single-chain antibody according to any one of claims 1 to 8 or the single-chain antibody prepared by the system according to any one of claims 12 to 13 for the diagnosis, prevention, and treatment of a disease or in the manufacture of a medicament for the diagnosis, prevention, and treatment of a disease; preferably, the disease is a tumor and/or cancer, wherein the tumor and/or cancer comprises lung cancer, breast cancer, esophageal cancer, gastric cancer, cervical cancer, colon cancer, rhabdomyosarcoma, liposarcoma, osteosarcoma, lymphoma, leukemia, liver cancer, cervical cancer, ovarian cancer, breast cancer, and other diseases; preferably, the disease is a disease caused by abnormalities in HER2, TNF-α, VEGF, PD-1, IL-4, IL-13, or CD38.
